(19)

Europäisches
Patentamt
European
Patent Office
Office européen
des brevets

(11) **EP 3 871 524 A1**

(12) **EUROPEAN PATENT APPLICATION**

(43) Date of publication:
**01.09.2021 Bulletin 2021/35**

(51) Int Cl.:
*A24F 40/50* (2020.01)    *A24F 40/57* (2020.01)

(21) Application number: **21159231.6**

(22) Date of filing: **25.02.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.02.2020   JP 2020029899**

(71) Applicant: **Japan Tobacco Inc.
Tokyo 105-6927 (JP)**

(72) Inventors:
• **Nakano, Takuma**
  **Tokyo, 130-0003 (JP)**
• **Kaihatsu, Yutaka**
  **Tokyo, 130-0003 (JP)**
• **Marubashi, Keiji**
  **Tokyo, 130-0003 (JP)**
• **Fujita, Hajime**
  **Tokyo, 130-0003 (JP)**

(74) Representative: **Hoffmann Eitle
Patent- und Rechtsanwälte PartmbB
Arabellastraße 30
81925 München (DE)**

(54) **POWER SUPPLY UNIT FOR AEROSOL INHALER AND AEROSOL INHALER**

(57)    A power supply unit for an aerosol inhaler that causes an aerosol generated from an aerosol source to pass through a flavor source to add a flavor component of the flavor source to the aerosol includes a power supply configured to be dischargeable to a first load configured to heat the aerosol source, and a processing device configured to cause determined power to be discharged from the power supply to the first load in response to a signal from a sensor configured to output the signal indicating an aerosol generation request. The processing device is configured to determine the power based on a time of discharging and a variable different from the time in discharging from the power supply to the first load in response to the signal before a previous time.

*FIG.5*

**Description**

TECHNICAL FIELD

The present invention relates to a power supply unit for an aerosol inhaler and an aerosol inhaler.

BACKGROUND ART

JP 2017-511703 T, WO 2018/017654, and WO 2018/020619 disclose an apparatus that can add a flavor component contained in a flavor source to an aerosol by passing the aerosol generated by heating a liquid through the flavor source, and cause a user to suck the aerosol containing the flavor component.

Apparatuses disclosed in JP 2017-511703 T, and WO 2018/017654 each include a heater that heats a liquid for aerosol generation and a heater that heats a flavor source.

WO 2018/020619 discloses that control of boosting an output voltage of a battery is performed such that an amount of aerosol generation does not decrease in conjunction with a decrease in the output voltage of the battery.

It is important that an aerosol inhaler can provide a large amount of a flavor component to the user in a stable manner in order to increase a commercial value. Patent Literatures 1 to 3 do not consider providing the flavor component to the user in a stable manner.

It is an object of the present invention to increase the commercial value of the aerosol inhaler.

SUMMARY OF INVENTION

According to an aspect of the present invention, there is provided a power supply unit for an aerosol inhaler that causes an aerosol generated from an aerosol source to pass through a flavor source to add a flavor component of the flavor source to the aerosol. The power supply unit includes, a power supply configured to be dischargeable to a first load configured to heat the aerosol source, and a processing device configured to cause determined power to be discharged from the power supply to the first load in response to a signal from a sensor configured to output the signal indicating an aerosol generation request. The processing device is configured to determine the power based on a time of discharging and a variable different from the time in discharging from the power supply to the first load in response to the signal before a previous time.

BRIEF DESCRIPTION OF DRAWINGS

Fig. 1 is a perspective view schematically showing a schematic configuration of an aerosol inhaler.
Fig. 2 is another perspective view of the aerosol inhaler of Fig. 1.
Fig. 3 is a cross-sectional view of the aerosol inhaler of Fig. 1.
Fig. 4 is a perspective view of a power supply unit of the aerosol inhaler of Fig. 1.
Fig. 5 is a schematic diagram showing a hardware configuration of the aerosol inhaler of Fig. 1.
Fig. 6 is a schematic diagram showing a modification of the hardware configuration of the aerosol inhaler of Fig. 1.
Fig. 7 is a diagram showing a specific example of the power supply unit shown in Fig. 5.
Fig. 8 is a diagram showing a specific example of a power supply unit shown in Fig. 6.
Fig. 9 is a flowchart for illustrating operations of the aerosol inhaler of Fig. 1.
Fig. 10 is a flowchart for illustrating the operations of the aerosol inhaler of Fig. 1.
Fig. 11 is a diagram showing a modification of the specific example of the power supply unit shown in Fig. 6.
Fig. 12 is a schematic diagram showing atomization power supplied to a first load in Step S17 of Fig. 10.
Fig. 13 is a schematic diagram showing atomization power supplied to the first load in Step S19 of Fig. 10.
Fig. 14 is a schematic diagram showing a first modification of the hardware configuration of the aerosol inhaler of Fig. 1.
Fig. 15 is a diagram showing a specific example of a power supply unit shown in Fig. 14.
Fig. 16 is a schematic diagram showing a second modification of the hardware configuration of the aerosol inhaler of Fig. 1.
Fig. 17 is a diagram showing a specific example of a power supply unit shown in Fig. 16.
Fig. 18 is a schematic diagram showing a third modification of the hardware configuration of the aerosol inhaler of Fig. 1.
Fig. 19 is a diagram showing a specific example of a power supply unit shown in Fig. 18.
Fig. 20 is a diagram showing a modification of the specific example of the power supply unit shown in Fig. 18.
Fig. 21 is a schematic diagram showing a fifth modification of the hardware configuration of the aerosol inhaler of Fig. 1.

Fig. 22 is a diagram showing a specific example of a power supply unit shown in Fig. 21.

Fig. 23 is a flowchart for illustrating a modification of the operations of the aerosol inhaler of Fig. 1.

Fig. 24 is a schematic diagram showing a change in atomization power when determination in Step S32 of Fig. 23 is YES.

DESCRIPTION OF EMBODIMENTS

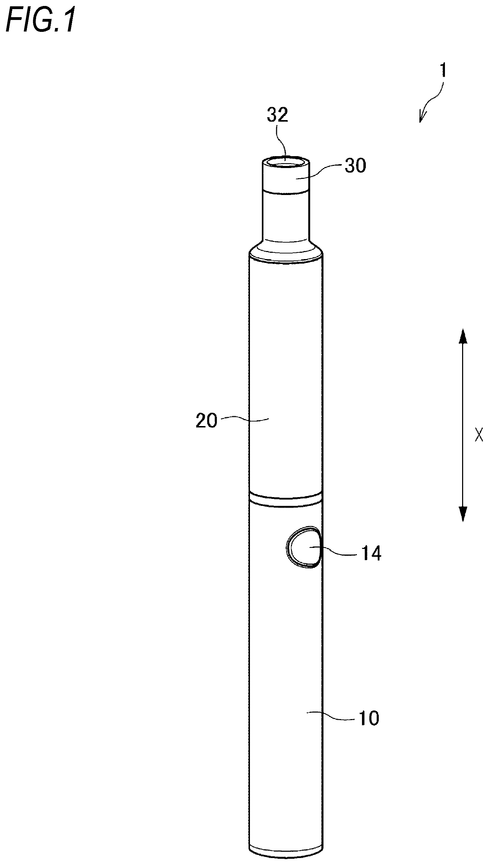

Hereinafter, an aerosol inhaler 1, which is an embodiment of an aerosol inhaler of the present invention, will be described with reference to Figs. 1 to 5.

(Aerosol Inhaler)

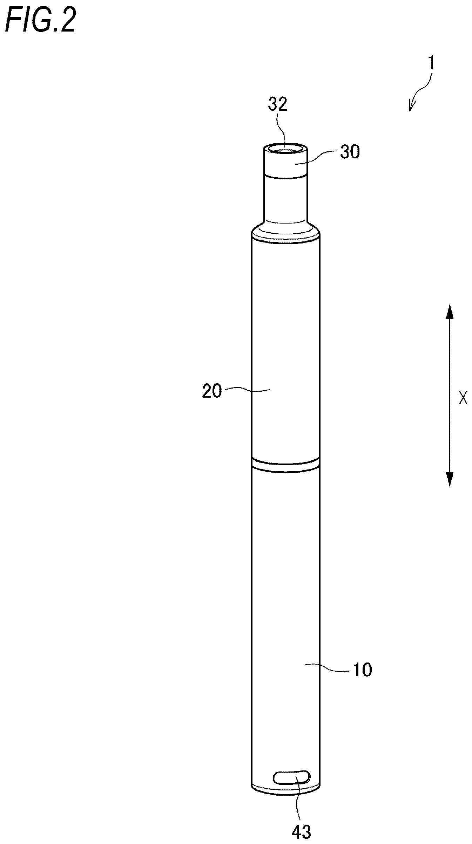

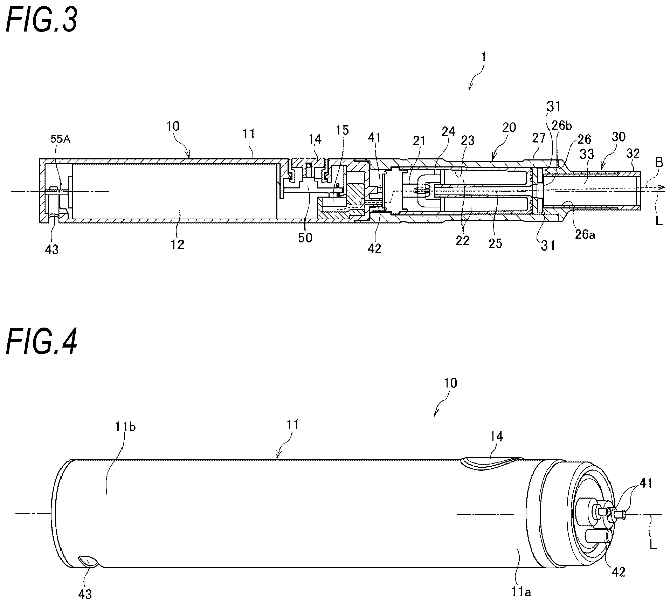

The aerosol inhaler 1 is a device for generating an aerosol to which a flavor component is added without burning and making it possible to suck the aerosol, and has a rod shape that extends along a predetermined direction (hereinafter, referred to as longitudinal direction X) as shown in Figs. 1 and 2. In the aerosol inhaler 1, a power supply unit 10, a first cartridge 20, and a second cartridge 30 are provided in this order along the longitudinal direction X. The first cartridge 20 is attachable to and detachable from (in other words, replaceable with respect to) the power supply unit 10. The second cartridge 30 is attachable to and detachable from (in other words, replaceable with respect to) the first cartridge 20. As shown in Fig. 3, the first cartridge 20 is provided with a first load 21 and a second load 31. As shown in Fig. 1, an overall shape of the aerosol inhaler 1 is not limited to a shape in which the power supply unit 10, the first cartridge 20, and the second cartridge 30 are lined up in a row. As long as the first cartridge 20 and the second cartridge 30 are replaceable with respect to the power supply unit 10, any shape such as a substantial box shape can be adopted. The second cartridge 30 may be attachable to and detachable from (in other words, replaceable with respect to) the power supply unit 10.

(Power Supply Unit)

As shown in Figs. 3, 4, and 5, the power supply unit 10 houses a power supply 12, a charging IC 55A, a micro controller unit (MCU) 50, a DC/DC converter 51, an intake sensor 15, a temperature detection element T1 including a voltage sensor 52 and a current sensor 53, and a temperature detection element T2 including a voltage sensor 54 and a current sensor 55 inside a cylindrical power supply unit case 11.

The power supply 12 is a rechargeable secondary battery, an electric double-layer capacitor, or the like, and is preferably a lithium-ion secondary battery. An electrolyte of the power supply 12 may be one of or a combination of a gel-like electrolyte, an electrolytic solution, a solid electrolyte, and an ionic liquid.

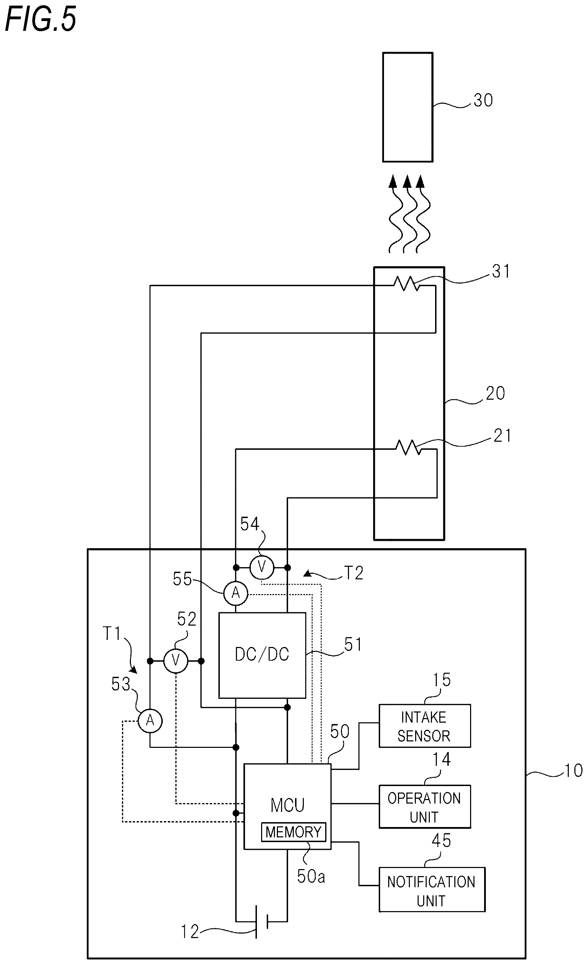

As shown in Fig. 5, the MCU 50 is connected to various sensor devices such as the intake sensor 15, the voltage sensor 52, the current sensor 53, the voltage sensor 54, and the current sensor 55, the DC/DC converter 51, an operation unit 14, and a notification unit 45, and performs various kinds of control of the aerosol inhaler 1.

Specifically, the MCU 50 is mainly configured with a processor, and further includes a memory 50a configured with a storage medium such as a random access memory (RAM) required for an operation of the processor and a read only memory (ROM) that stores various pieces of information. Specifically, the processor in the present description is an electric circuit in which circuit elements such as semiconductor elements are combined.

As shown in Fig. 4, discharging terminals 41 are provided on a top portion 11a positioned on one end side of the power supply unit case 11 in the longitudinal direction X (a first cartridge 20 side). The discharging terminal 41 is provided so as to protrude from an upper surface of the top portion 11a toward the first cartridge 20, and can be electrically connected to the first load 21 and the second load 31 of the first cartridge 20.

On the upper surface of the top portion 11a, an air supply unit 42 that supplies air to the first load 21 of the first cartridge 20 is provided in the vicinity of the discharging terminals 41.

A charging terminal 43 that can be electrically connected to an external power supply (not shown) is provided in a bottom portion 11b positioned on the other end side of the power supply unit case 11 in the longitudinal direction X (a side opposite to the first cartridge 20). The charging terminal 43 is provided in a side surface of the bottom portion 11b, and can be connected to, for example, a Universal Serial Bus (USB) terminal, a micro USB terminal, a Lightning (registered trademark) terminal, or the like.

The charging terminal 43 may be a power reception unit that can receive power transmitted from an external power supply in a wireless manner. In such a case, the charging terminal 43 (the power reception unit) may be configured with a power reception coil. A method for wireless power transfer may be an electromagnetic induction type or a magnetic resonance type. Further, the charging terminal 43 may be a power reception unit that can receive power transmitted from an external power supply without contact. As another example, the charging terminal 43 may be connected to the USB terminal, the micro USB terminal, or the Lightning terminal, and may include the power reception unit described above.

The power supply unit case 11 is provided with the operation unit 14 that can be operated by a user in the side surface of the top portion 11a so as to face a side opposite to the charging terminal 43. More specifically, the operation unit 14 and the charging terminal 43 have a point-symmetrical relationship with respect to an intersection between a straight line connecting the operation unit 14 and the charging terminal 43 and a center line of the power supply unit 10 in the longitudinal direction X. The operation unit 14 is configured with a button-type switch, a touch panel, or the like.

As shown in Fig. 3, the intake sensor 15 that detects a puff (suction) operation is provided in the vicinity of the operation unit 14. The power supply unit case 11 is provided with an air intake port (not shown) that takes outside air into the power supply unit case 11. The air intake port may be provided around the operation unit 14 or may be provided around the charging terminal 43.

The intake sensor 15 is configured to output a value of a pressure (an internal pressure) change in the power supply unit 10 caused by suction of the user through a suction port 32 described later. The intake sensor 15 is, for example, a pressure sensor that outputs an output value (for example, a voltage value or a current value) corresponding to an internal pressure that changes according to a flow rate of air sucked from the air intake port toward the suction port 32 (that is, a puff operation of the user). The intake sensor 15 may output an analog value or may output a digital value converted from the analog value.

The intake sensor 15 may incorporate a temperature sensor that detects a temperature of an environment (an outside air temperature) in which the power supply unit 10 is placed in order to compensate for a detected pressure. The intake sensor 15 may be configured with a condenser microphone or the like instead of the pressure sensor.

When a puff operation is performed and an output value of the intake sensor 15 is larger than a threshold, the MCU 50 determines that an aerosol generation request has been made, and then, when the output value of the intake sensor 15 is smaller than the threshold, the MCU 50 determines that the aerosol generation request has been ended. In the aerosol inhaler 1, when a period during which the aerosol generation request is made reaches a first default value $t_{upper}$ (for example, 2.4 seconds) for a purpose of preventing overheating of the first load 21 or the like, it is determined that the aerosol generation request has been ended regardless of an output value of the intake sensor 15. Accordingly, the output value of the intake sensor 15 is used as a signal indicating the aerosol generation request. Therefore, the intake sensor 15 constitutes a sensor that outputs an aerosol generation request.

Instead of the intake sensor 15, the aerosol generation request may be detected based on an operation of the operation unit 14. For example, when the user performs a predetermined operation on the operation unit 14 to start sucking aerosol, the operation unit 14 may be configured to output a signal indicating the aerosol generation request to the MCU 50. In this case, the operation unit 14 constitutes a sensor that outputs an aerosol generation request.

The charging IC 55A is disposed close to the charging terminal 43, and controls charging of power input from the charging terminal 43 to the power supply 12. The charging IC 55A may be disposed in the vicinity of the MCU 50.

(First Cartridge)

As shown in Fig. 3, the first cartridge 20 includes inside a cylindrical cartridge case 27, a reservoir 23 that stores an aerosol source 22, the first load 21 for atomizing the aerosol source 22, a wick 24 that draws the aerosol source from the reservoir 23 to the first load 21, an aerosol flow path 25 in which an aerosol generated by atomizing the aerosol source 22 flows toward the second cartridge 30, an end cap 26 that houses a part of the second cartridge 30, and the second load 31 provided in the end cap 26 and configured to heat the second cartridge 30.

The reservoir 23 is partitioned and formed so as to surround a periphery of the aerosol flow path 25 and stores the aerosol source 22. The reservoir 23 may house a porous body such as a resin web or cotton, and the aerosol source 22 may be impregnated in the porous body. The reservoir 23 may not house the porous body in the resin web or cotton and may only store the aerosol source 22. The aerosol source 22 contains a liquid such as glycerin, propylene glycol, or water.

The wick 24 is a liquid holding member that draws the aerosol source 22 from the reservoir 23 to the first load 21 by using a capillary phenomenon. The wick 24 is formed of, for example, glass fiber or porous ceramic.

The first load 21 atomizes the aerosol source 22 by heating the aerosol source 22 without burning by power supplied from the power supply 12 via the discharging terminals 41. The first load 21 is configured with an electric heating wire (a coil) wound at a predetermined pitch.

The first load 21 may be an element that can generate an aerosol by atomizing the aerosol source 22 by heating the aerosol source 22. The first load 21 is, for example, a heat generation element. Examples of the heat generation element include a heat generation resistor, a ceramic heater, and an induction heating type heater.

As the first load 21, a load in which a temperature and an electric resistance value have a correlation is used. As the first load 21, for example, a load having a positive temperature coefficient (PTC) characteristic in which the electric resistance value increases as the temperature increases is used.

The aerosol flow path 25 is provided on a downstream side of the first load 21 and on a center line L of the power supply unit 10. The end cap 26 includes a cartridge housing portion 26a that houses a part of the second cartridge 30, and a communication path 26b that causes the aerosol flow path 25 and the cartridge housing portion 26a to communicate with each other.

The second load 31 is embedded in the cartridge housing portion 26a. The second load 31 heats the second cartridge 30 (more specifically, a flavor source 33 included herein) housed in the cartridge housing portion 26a by the power supplied from the power supply 12 via the discharging terminals 41. The second load 31 is configured with, for example, an electric heating wire (a coil) wound at a predetermined pitch.

The second load 31 may be any element that can heat the second cartridge 30. The second load 31 is, for example, a heat generation element. Examples of the heat generation element include a heat generation resistor, a ceramic heater, and an induction heating type heater.

As the second load 31, a load in which a temperature and an electric resistance value have a correlation is used. As the second load 31, for example, a load having the PTC characteristic is used.

(Second Cartridge)

The second cartridge 30 stores the flavor source 33. When the second cartridge 30 is heated by the second load 31, the flavor source 33 is heated. The second cartridge 30 is detachably housed in the cartridge housing portion 26a provided in the end cap 26 of the first cartridge 20. An end portion of the second cartridge 30 on a side opposite to the first cartridge 20 side is the suction port 32 for the user. The suction port 32 is not limited to being integrally formed with the second cartridge 30 and may be attachable to and detachable from the second cartridge 30. Accordingly, the suction port 32 is configured separately from the power supply unit 10 and the first cartridge 20, so that the suction port 32 can be kept hygienic.

The second cartridge 30 adds a flavor component to an aerosol by passing, through the flavor source 33, the aerosol generated by atomizing the aerosol source 22 by the first load 21. As a raw material piece that constitutes the flavor source 33, cut tobacco or a molded body obtained by molding a tobacco raw material into a granular shape can be used. The flavor source 33 may be configured with a plant other than the tobacco (for example, mint, Chinese medicine, or herbs). A fragrance such as menthol may be added to the flavor source 33.

In the aerosol inhaler 1, the aerosol source 22 and the flavor source 33 can generate an aerosol to which a flavor component is added. That is, the aerosol source 22 and the flavor source 33 constitute an aerosol generation source that generates the aerosol.

The aerosol generation source of the aerosol inhaler 1 is a portion that the user replaces and uses. This portion is provided to the user, for example, as a set of one first cartridge 20 and one or a plurality of (for example, five) second cartridges 30. Therefore, in the aerosol inhaler 1, a replacement frequency of the power supply unit 10 is lowest, a replacement frequency of the first cartridge 20 is second lowest, and a replacement frequency of the second cartridge 30 is highest. Therefore, it is important to reduce a manufacturing cost of the first cartridge 20 and the second cartridge 30. The first cartridge 20 and the second cartridge 30 may be integrated into one cartridge.

In the aerosol inhaler 1 configured in this way, as indicated by an arrow B in Fig. 3, air that flows in from the intake port (not shown) provided in the power supply unit case 11 passes from the air supply unit 42 to a vicinity of the first load 21 of the first cartridge 20. The first load 21 atomizes the aerosol source 22 drawn from the reservoir 23 by the wick 24. An aerosol generated by atomization flows through the aerosol flow path 25 together with the air that flows in from the intake port, and is supplied to the second cartridge 30 via the communication path 26b. The aerosol supplied to the second cartridge 30 passes through the flavor source 33 to add a flavor component and is supplied to the suction port 32.

The aerosol inhaler 1 is provided with the notification unit 45 that notifies various pieces of information (see Fig. 5). The notification unit 45 may be configured with a light-emitting element, may be configured with a vibration element, or may be configured with a sound output element. The notification unit 45 may be a combination of two or more elements among the light-emitting element, the vibration element, and the sound output element. The notification unit 45 may be provided in any of the power supply unit 10, the first cartridge 20, and the second cartridge 30, but is preferably provided in the power supply unit 10. For example, a periphery of the operation unit 14 is translucent, and is configured to emit light by a light-emitting element such as an LED.

(Details of Power Supply Unit)

As shown in Fig. 5, in a state where the first cartridge 20 is mounted on the power supply unit 10, the DC/DC converter 51 is connected between the first load 21 and the power supply 12. The MCU 50 is connected between the DC/DC converter 51 and the power supply 12. In a state where the first cartridge 20 is mounted on the power supply unit 10, the second load 31 is connected to a connection node between the MCU 50 and the DC/DC converter 51. Accordingly, in the power supply unit 10, in a state where the first cartridge 20 is mounted, the second load 31 and a series circuit of the DC/DC converter 51 and the first load 21 are connected in parallel to the power supply 12.

The DC/DC converter 51 is a boosting circuit that can boost an input voltage, and is configured to be able to supply an input voltage or a voltage obtained by boosting the input voltage to the first load 21. Since power supplied to the first load 21 can be adjusted by the DC/DC converter 51, an amount of the aerosol source 22 to be atomized by the first load 21 can be controlled. As the DC/DC converter 51, for example, a switching regulator that converts an input voltage into a desired output voltage can be used by controlling on/off time of a switching element while monitoring the output voltage. When the switching regulator is used as the DC/DC converter 51, the input voltage can be output as it is without boosting by controlling the switching element.

The processor of the MCU 50 is configured to be able to acquire a temperature of the flavor source 33 in order to control discharging to the second load 31, which will be described later. The processor of the MCU 50 is preferably configured to be able to acquire a temperature of the first load 21. The temperature of the first load 21 can be used to prevent overheating of the first load 21 and the aerosol source 22 and to highly control the amount of the aerosol source 22 to be atomized by the first load 21.

The voltage sensor 52 measures and outputs a value of a voltage applied to the second load 31. The current sensor 53 measures and outputs a value of a current that flows through the second load 31. An output of the voltage sensor 52 and an output of the current sensor 53 are input to the MCU 50. The processor of the MCU 50 acquires a resistance value of the second load 31 based on the output of the voltage sensor 52 and the output of the current sensor 53, and acquires a temperature of the second load 31 according to the resistance value. The temperature of the second load 31 does not exactly coincide with a temperature of the flavor source 33 heated by the second load 31, but can be regarded as substantially the same as the temperature of the flavor source 33. Therefore, the temperature detection element T1 constitutes a temperature detection element for detecting the temperature of the flavor source 33.

If a constant current flows to the second load 31 when the resistance value of the second load 31 is acquired, the current sensor 53 is unnecessary in the temperature detection element T1. Similarly, if a constant voltage is applied to the second load 31 when the resistance value of the second load 31 is acquired, the voltage sensor 52 is unnecessary in the temperature detection element T1.

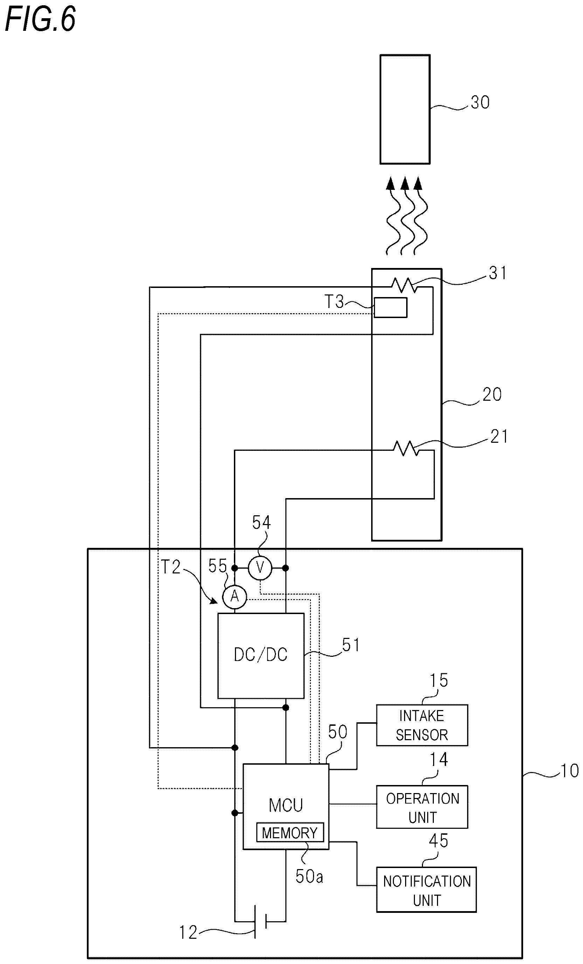

As shown in Fig. 6, instead of the temperature detection element T1, the first cartridge 20 may be provided with a temperature detection element T3 for detecting a temperature of the second cartridge 30. The temperature detection element T3 is configured with, for example, a thermistor disposed in the vicinity of the second cartridge 30. In the configuration of Fig. 6, the processor of the MCU 50 acquires the temperature of the second cartridge 30 (in other words, the flavor source 33) based on an output of the temperature detection element T3.

As shown in Fig. 6, since the temperature of the second cartridge 30 (the flavor source 33) is acquired by using the temperature detection element T3, compared with acquiring the temperature of the flavor source 33 by using the temperature detection element T1 in Fig. 5, the temperature of the flavor source 33 can be more accurately acquired. The temperature detection element T3 may be mounted on the second cartridge 30. According to the configuration shown in Fig. 6 in which the temperature detection element T3 is mounted on the first cartridge 20, a manufacturing cost of the second cartridge 30 having highest replacement frequency in the aerosol inhaler 1 can be reduced.

As shown in Fig. 5, when the temperature of the second cartridge 30 (the flavor source 33) is acquired by using the temperature detection element T1, the temperature detection element T1 can be provided in the power supply unit 10 having lowest replacement frequency in the aerosol inhaler 1. Therefore, manufacturing costs of the first cartridge 20 and the second cartridge 30 can be reduced.

The voltage sensor 54 measures and outputs a value of a voltage applied to the first load 21. The current sensor 55 measures and outputs a value of a current that flows through the first load 21. An output of the voltage sensor 54 and an output of the current sensor 55 are input to the MCU 50. The processor of the MCU 50 acquires a resistance value of the first load 21 based on the output of the voltage sensor 54 and the output of the current sensor 55, and acquires a temperature of the first load 21 according to the resistance value. If a constant current flows to the first load 21 when the resistance value of the first load 21 is acquired, the current sensor 55 is unnecessary in the temperature detection element T2. Similarly, if a constant voltage is applied to the first load 21 when the resistance value of the first load 21 is acquired, the voltage sensor 54 is unnecessary in the temperature detection element T2.

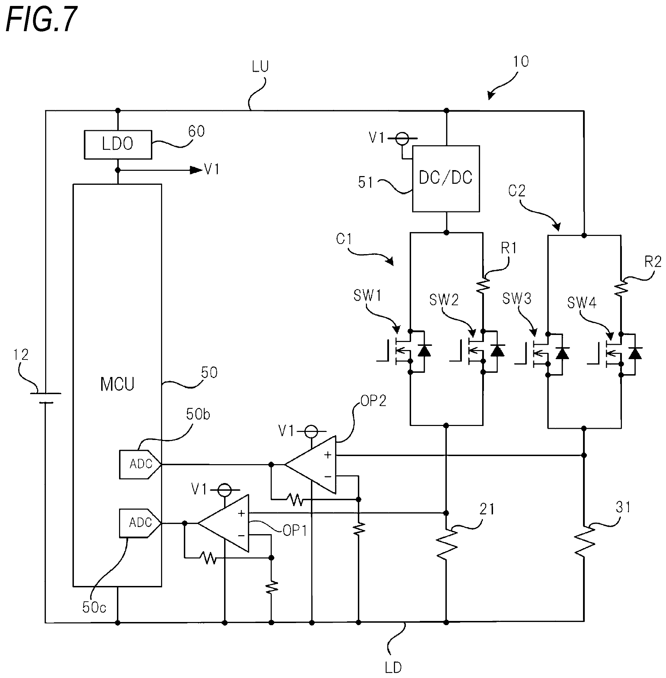

Fig. 7 is a diagram showing a specific example of the power supply unit 10 shown in Fig. 5. Fig. 7 shows a specific example of a configuration in which the temperature detection element T1 does not include the current sensor 53 and the temperature detection element T2 does not include the current sensor 55.

As shown in Fig. 7, the power supply unit 10 includes the power supply 12, the MCU 50, a low drop out (LDO) regulator 60, a switch SW1, a parallel circuit C1 including a series circuit of a resistance element R1 and a switch SW2 connected in parallel to the switch SW1, a switch SW3, a parallel circuit C2 including a series circuit of a resistance element R2 and a switch SW4 connected in parallel to the switch SW3, an operational amplifier OP1 and analog-to-digital converter (hereinafter, referred to as ADC) 50c that constitute the voltage sensor 54, and an operational amplifier OP2 and an ADC 50b that constitute the voltage sensor 52.

The resistance element described in the present description may be an element having a fixed electric resistance value, for example, a resistor, a diode, or a transistor. In the example of Fig. 7, the resistance element R1 and the resistance element R2 are resistors.

The switch described in the present description is a switching element such as a transistor that switches between interruption and conduction of a wiring path. In the example of Fig. 7, the switches SW1 to SW4 are transistors.

The LDO regulator 60 is connected to a main positive bus LU connected to a positive electrode of the power supply 12. The MCU 50 is connected to the LDO regulator 60 and a main negative bus LD connected to a negative electrode of the power supply 12. The MCU 50 is also connected to the switches SW1 to SW4, and controls opening and closing of these switches. The LDO regulator 60 reduces a voltage from the power supply 12 and outputs the reduced voltage. An output voltage VI of the LDO regulator 60 is also used as respective operation voltages of the MCU 50, the DC/DC converter 51, the operational amplifier OP1, and the operational amplifier OP2.

The DC/DC converter 51 is connected to the main positive bus LU. The first load 21 is connected to the main negative bus LD. The parallel circuit C1 is connected to the DC/DC converter 51 and the first load 21.

The parallel circuit C2 is connected to the main positive bus LU. The second load 31 is connected to the parallel circuit C2 and the main negative bus LD.

A non-inverting input terminal of the operational amplifier OP1 is connected to a connection node between the parallel circuit C1 and the first load 21. An inverting input terminal of the operational amplifier OP1 is connected to an output terminal of the operational amplifier OP1 and the main negative bus LD via the resistance element.

A non-inverting input terminal of the operational amplifier OP2 is connected to a connection node between the parallel circuit C2 and the second load 31. An inverting input terminal of the operational amplifier OP2 is connected to an output terminal of the operational amplifier OP2 and the main negative bus LD via the resistance element.

The ADC 50c is connected to the output terminal of the operational amplifier OP1. The ADC 50b is connected to the output terminal of the operational amplifier OP2. The ADC 50c and the ADC 50b may be provided at an outside of the MCU 50.

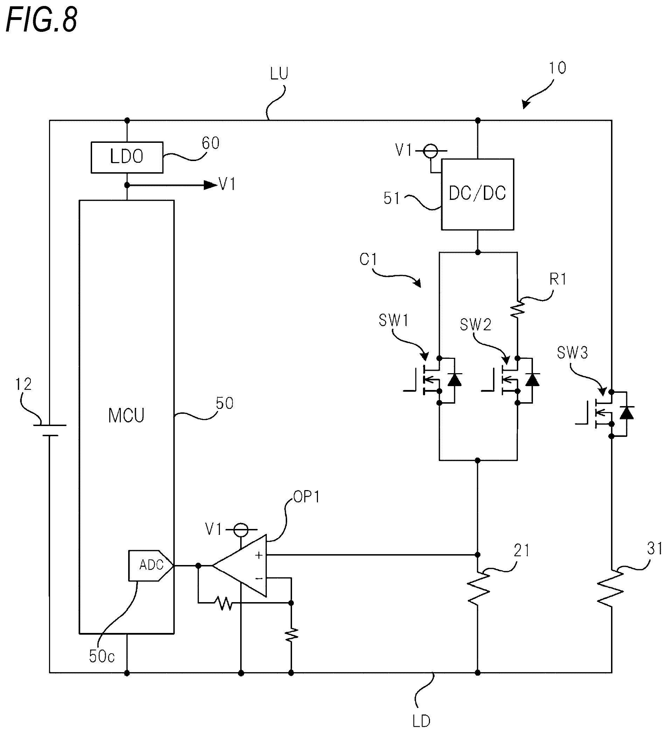

Fig. 8 is a diagram showing a specific example of the power supply unit 10 shown in Fig. 6. Fig. 8 shows a specific example of a configuration in which the temperature detection element T2 does not include the voltage sensor 54. A circuit shown in Fig. 8 has the same configuration as that of Fig. 7 except that the operational amplifier OP2, the ADC 50b, the resistance element R2, and the switch SW4 are eliminated.

(MCU)

Next, functions of the MCU 50 will be described. The MCU 50 includes a temperature detection unit, a power control unit, and a notification control unit as a functional block implemented by executing a program stored in a ROM by the processor.

The temperature detection unit acquires a temperature of the flavor source 33 based on an output of the temperature detection element T1 (or the temperature detection element T3). Further, the temperature detection unit acquires a temperature of the first load 21 based on an output of the temperature detection element T2.

In a case of the circuit example shown in Fig. 7, the temperature detection unit controls the switch SW1, the switch SW3, and the switch SW4 to be in an interruption state, acquires an output value of the ADC 50c (a value of a voltage applied to the first load 21) in a state where the switch SW2 is controlled to be in a conductive state, and acquires a temperature of the first load 21 based on the output value.

The non-inverting input terminal of the operational amplifier OP1 may be connected to a terminal of the resistance element R1 on a DC/DC converter 51 side, and the inverting input terminal of the operational amplifier OP1 may be connected to a terminal of the resistance element R1 on a switch SW2 side. In this case, the temperature detection unit can control the switch SW1, the switch SW3, and the switch SW4 to be in an interruption state, acquire an output value of the ADC 50c (a value of a voltage applied to the resistance element R1) in a state where the switch SW2 is controlled to be in a conductive state, and acquire a temperature of the first load 21 based on the output value.

In the case of the circuit example shown in Fig. 7, the temperature detection unit controls the switch SW1, the switch SW2, and the switch SW3 to be in an interruption state, acquires an output value of the ADC 50b (a value of a voltage applied to the second load 31) in a state where the switch SW4 is controlled to be in a conductive state, and acquires a temperature of the second load 31 as a temperature of the flavor source 33 based on the output value.

The non-inverting input terminal of the operational amplifier OP2 may be connected to a terminal of the resistance element R2 on a main positive bus LU side, and the inverting input terminal of the operational amplifier OP2 may be connected to a terminal of the resistance element R2 on a switch SW4 side. In this case, the temperature detection unit can control the switch SW1, the switch SW2, and the switch SW3 to be in an interruption state, acquire an output value of the ADC 50b (a value of a voltage applied to the resistance element R2) in a state where the switch SW4 is controlled to be in a conductive state, and acquire a temperature of the second load 31 as a temperature of the flavor source 33 based on the output value.

In a case of the circuit example shown in Fig. 8, the temperature detection unit controls the switch SW1 and the switch SW3 to be in an interruption state, acquires an output value of the ADC 50c (a value of a voltage applied to the first load 21) in a state where the switch SW2 is controlled to be in a conductive state, and acquires a temperature of the first load 21 based on the output value.

The notification control unit controls the notification unit 45 so as to notify various pieces of information. For example, the notification control unit controls the notification unit 45 so as to give a notification that prompts replacement of the second cartridge 30 in response to detection of a replacement timing of the second cartridge 30. The notification control unit is not limited to the notification that prompts the replacement of the second cartridge 30, and may give a notification that prompts replacement of the first cartridge 20, a notification that prompts replacement of the power supply 12, a notification that prompts charging of the power supply 12, and the like.

The power control unit controls discharging from the power supply 12 to at least the first load 21 (discharging required for heating a load) of the first load 21 and the second load 31 in response to a signal indicating the aerosol generation request output from the intake sensor 15.

In the case of the circuit example shown in Fig. 7, the power control unit controls the switch SW2, the switch SW3, and the switch SW4 to be in an interruption state, and controls the switch SW1 to be in a conductive state, so that discharging is performed from the power supply 12 to the first load 21 to atomize the aerosol source 22. Further, the power control unit controls the switch SW1, the switch SW2, and the switch SW4 to be in an interruption state and controls the switch SW3 to be in a conductive state, so that discharging is performed from the power supply 12 to the second load 31 to heat the flavor source 33.

In the case of the circuit example shown in Fig. 8, the power control unit controls the switch SW2 and the switch SW3 to be in an interruption state and controls the switch SW1 to be in a conductive state, so that discharging is performed from the power supply 12 to the first load 21 to atomize the aerosol source 22. Further, the power control unit controls the switch SW1 and the switch SW2 to be in an interruption state and controls the switch SW3 to be in a conductive state, so that discharging is performed from the power supply 12 to the second load 31 to heat the flavor source 33.

Accordingly, in the aerosol inhaler 1, the flavor source 33 can be heated by discharging to the second load 31. In order to increase an amount of the flavor component added to the aerosol, it is experimentally found that increasing an amount of an aerosol generated from the aerosol source 22 and increasing the temperature of the flavor source 33 are effective.

Therefore, based on information on the temperature of the flavor source 33, the power control unit controls discharging for heating from the power supply 12 to the first load 21 and the second load 31 such that a unit flavor amount (an amount of a flavor component $W_{flavor}$ described below), which is an amount of a flavor component added to an aerosol generated for each aerosol generation request, converges to a target amount. The target amount is an appropriately determined value. For example, a target range of the unit flavor amount may be appropriately determined, and a median value of the target range may be determined as the target amount. Accordingly, by converging the unit flavor amount (the amount of the flavor component $W_{flavor}$) to the target amount, it is possible to also converge the unit flavor amount to a target range having a certain width. A weight may be used as a unit of the unit flavor amount, the amount of the flavor component $W_{flavor}$, and the target amount.

Based on an output of the temperature detection element T1 (or the temperature detection element T3) that outputs information on a temperature of the flavor source 33, the power control unit controls discharging for heating from the power supply 12 to the second load 31 such that the temperature of the flavor source 33 converges to a target temperature (a target temperature $T_{cap\_target}$ described below).

(Various Parameters Used For Aerosol Generation)

Hereinafter, various parameters and the like used for discharging control for aerosol generation will be described before moving on to description of a specific operation of the MCU 50.

A weight [mg] of an aerosol that is generated in the first cartridge 20 and passes through the flavor source 33 by one suction operation by the user is referred to as an aerosol weight $W_{aerosol}$. Power required to be supplied to the first load 21 for generating the aerosol is referred to as atomization power $P_{liquid}$. The aerosol weight $W_{aerosol}$ is proportional to the atomization power $P_{liquid}$ and a supply time $t_{sense}$ of the atomization power $P_{liquid}$ to the first load 21 (in other words, a time when the first load 21 is energized or a time when a puff is performed assuming that the aerosol source 22 is sufficiently present. Therefore, the aerosol weight $W_{aerosol}$ can be modeled by the following Equation (1). The $\alpha$ in Equation (1) is a coefficient obtained experimentally. The first default value $t_{upper}$ described above is set as an upper limit value for the supply time $t_{sense}$. Further, the following Equation (1) may be replaced with Equation (1A). In Equation (1A), an intercept b having a positive value is introduced into Equation (1), which is an item that can be optionally introduced in consideration of a fact that a part of the atomization power $P_{liquid}$ is used for increasing a temperature of the aerosol source 22 that occurs before atomization in the aerosol source 22. The intercept b can also be experimentally obtained.

$$W_{aerosol} \equiv \alpha \times P_{liquid} \times t_{sense} \ (1)$$

$$W_{aerosol} \equiv \alpha \times P_{liquid} \times t_{sense} - b \ (1A)$$

A weight [mg] of a flavor component contained in the flavor source 33 in a state where suction is performed $n_{puff}$ (the $n_{puff}$ is a natural number equal to or larger than 0) times is referred to as a flavor component remaining amount $W_{capsule} (n_{puff})$. A remaining amount of a flavor component ($W_{capsule} (n_{puff} = 0)$) contained in the flavor source 33 of the second cartridge 30 in a new product state is also referred to as $W_{initial}$. Information on a temperature of the flavor source 33 is referred to as a capsule temperature parameter $T_{capsule}$. A weight [mg] of a flavor component added to an aerosol that passes through the flavor source 33 by one suction operation by the user is referred to as the amount of the flavor component $W_{flavor}$. The information on the temperature of the flavor source 33 is, for example, a temperature of the flavor source 33 or a temperature of the second load 31 acquired based on an output of the temperature detection element T1 (or the temperature detection element T3).

It is experimentally found that the amount of the flavor component $W_{flavor}$ depends on the flavor component remaining amount $W_{capsule} (n_{puff})$, the capsule temperature parameter $T_{capsule}$, and the aerosol weight $W_{aerosol}$. Therefore, the amount of the flavor component $W_{flavor}$ can be modeled by the following Equation (2).

$$W_{flavor} = \beta \times \{W_{capsule} (n_{puff}) \times T_{capsule}\} \times \gamma \times W_{aerosol} \ (2)$$

Every time a single suction is performed, the flavor component remaining amount $W_{capsule} (n_{puff})$ decreases by the amount of the flavor component $W_{flavor}$. Therefore, the flavor component remaining amount $W_{capsule} (n_{puff})$ can be modeled by the following Equation (3).
[Formula 1]

$$W_{capsule}\left(n_{puff}\right) = W_{initial} - \delta \cdot \sum_{i=1}^{n_{puff}} W_{flavor}(i) \ \cdots (3)$$

The $\beta$ in Equation (2) is a coefficient indicating a ratio of how much of the flavor component contained in the flavor source 33 is added to an aerosol in one suction, and is experimentally obtained. The $\gamma$ in Equation (2) and the $\delta$ in Equation (3) are experimentally obtained coefficients, respectively. The capsule temperature parameter $T_{capsule}$ and the flavor component remaining amount $W_{capsule} (n_{puff})$ can fluctuate during a period in which one suction is performed, but in this model, the $\gamma$ and the $\delta$ are introduced in order to handle the capsule temperature parameter $T_{capsule}$ and the flavor component remaining amount $W_{capsule} (n_{puff})$ as constant values.

(Operations of Aerosol Inhaler)

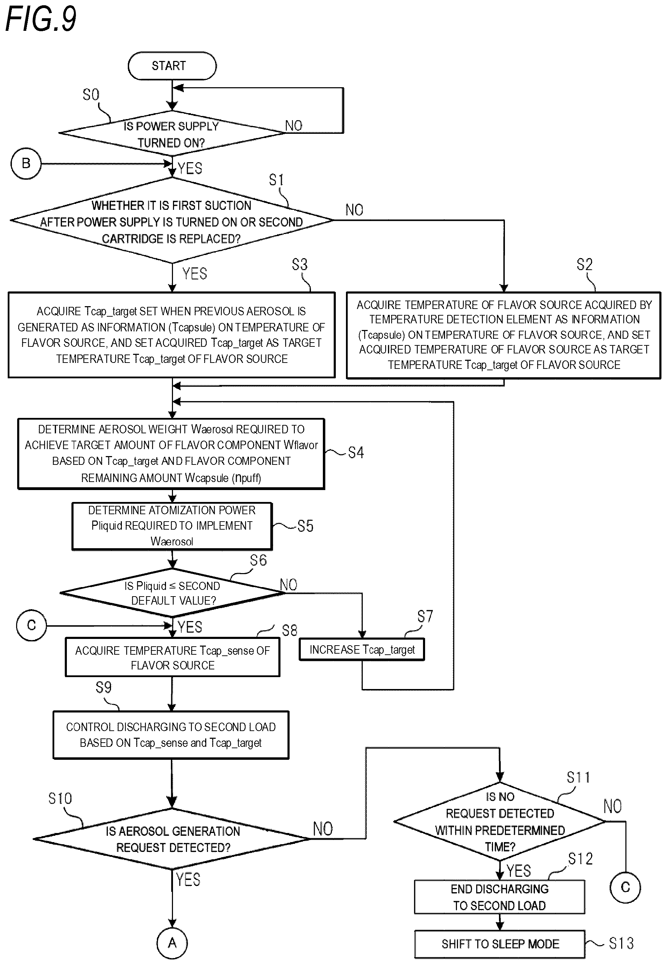

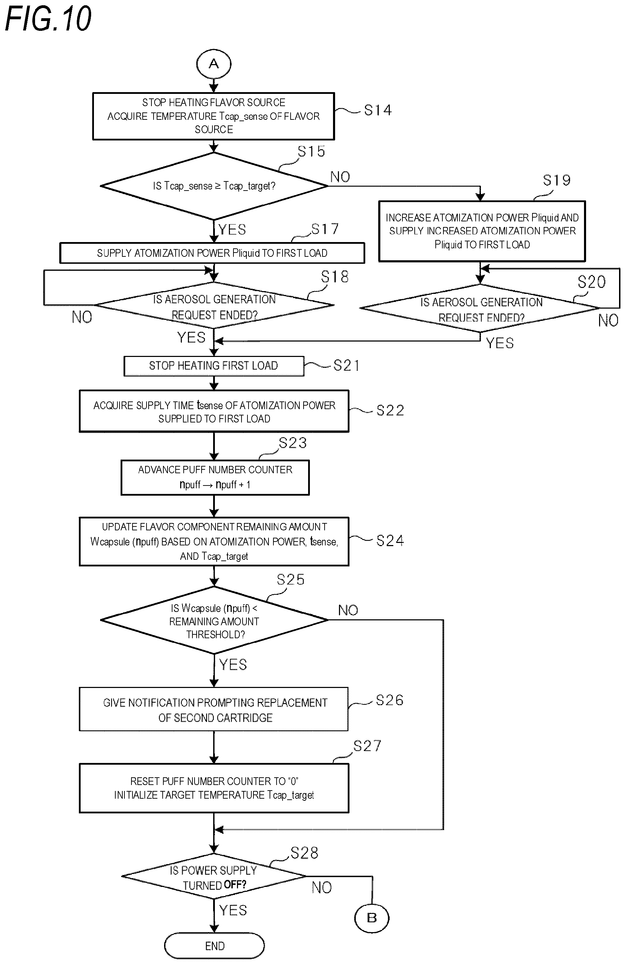

Figs. 9 and 10 are flowcharts for illustrating operations of the aerosol inhaler 1 in Fig. 1. When a power supply of the aerosol inhaler 1 is turned on by an operation on the operation unit 14 or the like (Step S0: YES), the MCU 50 determines whether an aerosol is generated after the power supply is turned on or after the second cartridge 30 is replaced (whether suction by the user is performed even once) (Step S1).

For example, every time suction (the aerosol generation request) is performed, the MCU 50 incorporates a puff number counter that counts up the $n_{puff}$ from an initial value (for example, 0). A count value of the puff number counter is stored in the memory 50a. The count value is referred to, so that the MCU 50 determines whether a state is after suction has been performed even once.

When it is a first suction after the power supply is turned on, or it is a timing before the first suction after the second cartridge 30 is replaced (Step S1: NO), the flavor source 33 has not yet been heated or has not been heated for a while, and a temperature of the flavor source 33 is likely to depend on an external environment. Therefore, in this case, the MCU 50 acquires a temperature of the flavor source 33 acquired based on an output of the temperature detection element T1 (or the temperature detection element T3) as the capsule temperature parameter $T_{capsule}$, sets the acquired temperature of the flavor source 33 as a target temperature $T_{cap\_target}$ of the flavor source 33, and stores the temperature of the flavor source 33 in the memory 50a (Step S2).

In a state where the determination in Step S1 is NO, the temperature of the flavor source 33 is likely to be close to an outside air temperature or a temperature of the power supply unit 10. Therefore, in Step S2, as a modification, the outside air temperature or the temperature of the power supply unit 10 may be acquired as the capsule temperature parameter $T_{capsule}$, and used as the target temperature $T_{cap\_target}$.

The outside air temperature is preferably acquired from, for example, a temperature sensor incorporated in the intake sensor 15. The temperature of the power supply unit 10 is preferably acquired from, for example, a temperature sensor incorporated in the MCU 50 in order to manage a temperature inside the MCU 50. In this case, both the temperature sensor incorporated in the intake sensor 15 and the temperature sensor incorporated in the MCU 50 function as elements that output the information on the temperature of the flavor source 33.

In the aerosol inhaler 1, as described above, discharging from the power supply 12 to the second load 31 is controlled such that the temperature of the flavor source 33 converges to the target temperature $T_{cap\_target}$. Therefore, the temperature of the flavor source 33 is likely to be close to the target temperature $T_{cap\_target}$ after the suction is performed even once after the power supply is turned on or after the second cartridge 30 is replaced. Therefore, in this case (Step S1: YES), the MCU 50 acquires the target temperature $T_{cap\_target}$ that is used for previously generating a aerosol and is stored in the memory 50a as the capsule temperature parameter $T_{capsule}$, and sets the target temperature $T_{cap\_target}$ described above as it is as the target temperature $T_{cap\_target}$ (Step S3). In this case, the memory 50a functions as an element that outputs the information on the temperature of the flavor source 33.

In Step S3, the MCU 50 may acquire the temperature of the flavor source 33 acquired based on the output of the temperature detection element T1 (or the temperature detection element T3) as the capsule temperature parameter $T_{capsule}$, and may set the acquired temperature of the flavor source 33 as the target temperature $T_{cap\_target}$ of the flavor source 33. Accordingly, the capsule temperature parameter $T_{capsule}$ can be more accurately acquired.

After Step S2 or Step S3, based on the set target temperature $T_{cap\_target}$ and the flavor component remaining amount $W_{capsule}$ ($n_{puff}$) of the flavor source 33 at a present time, the MCU 50 determines the aerosol weight $W_{aerosol}$ required to achieve the target amount of the flavor component $W_{flavor}$ by calculation of Equation (4) (Step S4). Equation (4) is obtained by modifying Equation (2) in which the $T_{capsule}$ is set as the $T_{cap\_target}$.

$$W_{aerosol} = W_{flavor}/ [\beta \times \{W_{capsule} (n_{puff}) \times T_{cap\_target}\} \times \gamma] (4)$$

Next, the MCU 50 determines the atomization power $P_{liquid}$ required to implement the aerosol weight $W_{aerosol}$ determined in Step S4 by calculation of Equation (1) in which the $t_{sense}$ is set as the first default value $t_{upper}$ (Step S5).

A table in which the atomization power $P_{liquid}$ and a combination of the target temperature $T_{cap\_target}$ and the flavor component remaining amount $W_{capsule}$ ($n_{puff}$) are associated with each other may be stored in the memory 50a of the MCU 50. And the MCU 50 may use the table to determine the atomization power $P_{liquid}$. Accordingly, the atomization power $P_{liquid}$ can be determined at a high speed and low power consumption.

Next, the MCU 50 determines whether the atomization power $P_{liquid}$ determined in Step S5 is equal to or smaller than a second default value (Step S6). The second default value is a maximum value of power that can be discharged from the power supply 12 to the first load 21 at that time, or a value obtained by subtracting a predetermined value from the maximum value.

When discharging from the power supply 12 to the first load 21, a current that flows through the first load 21 and a voltage of the power supply 12 are referred to as I and $V_{LIB}$, respectively. An upper limit value of a boosting rate of the DC/DC converter 51 is referred to as $\eta_{upper}$. An upper limit value of an output voltage of the DC/DC converter 51 is referred to as $P_{DC/DC\_upper}$. The second default value is referred to as $P_{upper}$. An electric resistance value of the first load 21 in a state where a temperature of the first load 21 reaches a boiling point temperature of the aerosol source 22 is referred to as $R_{HTR}(T_{HTR}=T_{B.P})$. With these references, the second default value $P_{upper}$ can be expressed by the following Equation (5).

[Formula 2]

$$P_{upper} = I \cdot V_{LIB} = MIN\left(\frac{(\eta_{upper} \cdot V_{LIB})^2}{R_{HTR}(T_{HTR} = T_{B.P.})} \quad P_{DC/DC\_upper}\right) - \Delta \cdots (5)$$

In Equation (5), $\Delta = 0$ is an ideal value of the second default value $P_{upper}$. However, in an actual circuit, it is necessary to consider a resistance component of a lead wire connected to the first load 21 and a resistance component and the like other than a resistance component connected to the first load 21. Therefore, in order to provide a certain margin, the adjustment value $\Delta$ is introduced in Equation (5).

In the aerosol inhaler 1, the DC/DC converter 51 is not essential and can be omitted. When the DC/DC converter 51 is omitted, the second default value $P_{upper}$ can be expressed by the following Equation (6).
[Formula 3]

$$P_{upper} = I \cdot V_{LIB} = \frac{V_{LIB}^2}{R_{HTR}(T_{HTR} = T_{B.P.})} - \Delta \cdots (6)$$

When the atomization power $P_{liquid}$ determined in Step S5 is larger than the second default value $P_{upper}$ (Step S6: NO), the MCU 50 increases the target temperature $T_{cap\_target}$ by a predetermined amount and returns the processing to Step S4. As can be seen from Equation (4), by increasing the target temperature $T_{cap\_target}$, the aerosol weight $W_{aerosol}$ required to achieve the target amount of the flavor component $W_{flavor}$ can be reduced, and as a result, the atomization power $P_{liquid}$ determined in Step S5 can be reduced. The MCU 50 repeats Steps S4 to S7, so that the determination in Step S6 determined initially as NO is determined as YES, and the processing can be shifted to Step S8.

When the atomization power $P_{liquid}$ determined in Step S5 is equal to or smaller than the second default value $P_{upper}$ (Step S6: YES), the MCU 50 acquires the temperature $T_{cap\_sense}$ of the flavor source 33 at a present time based on the output of the temperature detection element T1 (or the temperature detection element T3) (Step S8).

Then, the MCU 50 controls discharging to the second load 31 for heating the second load 31 based on the temperature $T_{cap\_sense}$ and the target temperature $T_{cap\_target}$ (Step S9). Specifically, the MCU 50 supplies power to the second load 31 by proportional-integral-differential (PID) control or ON/OFF control such that the temperature $T_{cap\_sense}$ converges to the target temperature $T_{cap\_target}$.

In the PID control, a difference between the temperature $T_{cap\_sense}$ and the target temperature $T_{cap\_target}$ is fed back, and based on the feedback result, power control is performed such that the temperature $T_{cap\_sense}$ converges to the target temperature $T_{cap\_target}$. According to the PID control, the temperature $T_{cap\_sense}$ can converge to the target temperature $T_{cap\_target}$ with high accuracy. The MCU 50 may use proportional (P) control or proportional-integral (PI) control instead of the PID control.

The ON/OFF control is control in which when the temperature $T_{cap\_sense}$ is lower than the target temperature $T_{cap\_target}$, power is supplied to the second load 31, and when the temperature $T_{cap\_sense}$ is equal to or higher than the target temperature $T_{cap\_target}$, the power supply to the second load 31 is stopped until the temperature $T_{cap\_sense}$ is lower than the target temperature $T_{cap\_target}$. According to the ON/OFF control, the temperature of the flavor source 33 can be increased faster than that in the PID control. Therefore, it is possible to increase a possibility that the temperature $T_{cap\_sense}$ reaches the target temperature $T_{cap\_target}$ at a stage before the aerosol generation request described later is detected. The target temperature $T_{cap\_target}$ may have hysteresis.

After Step S9, the MCU 50 determines whether there is the aerosol generation request (Step S10). When detecting no aerosol generation request (Step S10: NO), the MCU 50 determines, in Step S11, a length of time during which the aerosol generation request is not made (hereinafter, referred to as non-operation time). Then, when the non-operation time reaches a predetermined time (Step S11: YES), the MCU 50 ends discharging to the second load 31 (Step S12), and shifts to a sleep mode in which power consumption is reduced (Step S13). When the non-operation time is less than a predetermined time (Step S11: NO), the MCU 50 shifts the processing to Step S8.

When detecting the aerosol generation request (Step S10: YES), the MCU 50 ends discharging to the second load 31, and acquires the temperature $T_{cap\_sense}$ of the flavor source 33 at that time based on the output of the temperature detection element T1 (or the temperature detection element T3) (Step S14). Then, the MCU 50 determines whether the temperature $T_{cap\_sense}$ acquired in Step S14 is equal to or higher than the target temperature $T_{cap\_target}$ (Step S15).

When the temperature $T_{cap\_sense}$ is lower than the target temperature $T_{cap\_target}$ (Step S15: NO), the MCU 50 supplies atomization power $P_{liquid}'$ (second power), which is obtained by increasing the atomization power $P_{liquid}$ (first power) determined in Step S5 by a predetermined amount, to the first load 21 to start heating the first load 21 (Step S19). The increase in the power here is determined within a range in which the atomization power $P_{liquid}'$ is not larger than the ideal value of the second default value $P_{upper}$ described above.

For example, in Steps S17 and S19, it is assumed that the atomization power to be supplied to the first load 21 (the power determined by the MCU 50) is a value that can be discharged from the power supply 12 to the first load 21 without boosting by the DC/DC converter 51 (in other words, even when the boosting by the DC/DC converter 51 is stopped). In this case, it is preferable that the MCU 50 controls the switching element of the DC/DC converter 51, and supplies a voltage from the power supply 12 to the first load 21 without boosting the voltage such that the DC/DC converter 51 outputs the input voltage as it is. As an example, when the DC/DC converter 51 is a boosting type switching regulator, the DC/DC converter 51 can output the input voltage as it is by keeping the switching element OFF. Accordingly, a power loss due to boosting of the DC/DC converter 51 can be reduced, and power consumption can be suppressed.

On the other hand, for example, in Steps S17 and S19, it is assumed that the atomization power to be supplied to the first load 21 is a value that cannot be discharged from the power supply 12 to the first load 21 without boosting by the DC/DC converter 51. In this case, the MCU 50 may control the switching element of the DC/DC converter 51, boost the voltage from the power supply 12, and supply the boosted voltage to the first load 21 such that the DC/DC converter 51 boosts and outputs the input voltage. Accordingly, it is possible to supply the required power to the first load 21 while suppressing power consumption. As is clear from Equations (5) and (6), if the DC/DC converter 51 is provided, power that can be discharged from the power supply 12 to the first load 21 can be increased. Therefore, the unit flavor amount can be made more stable.

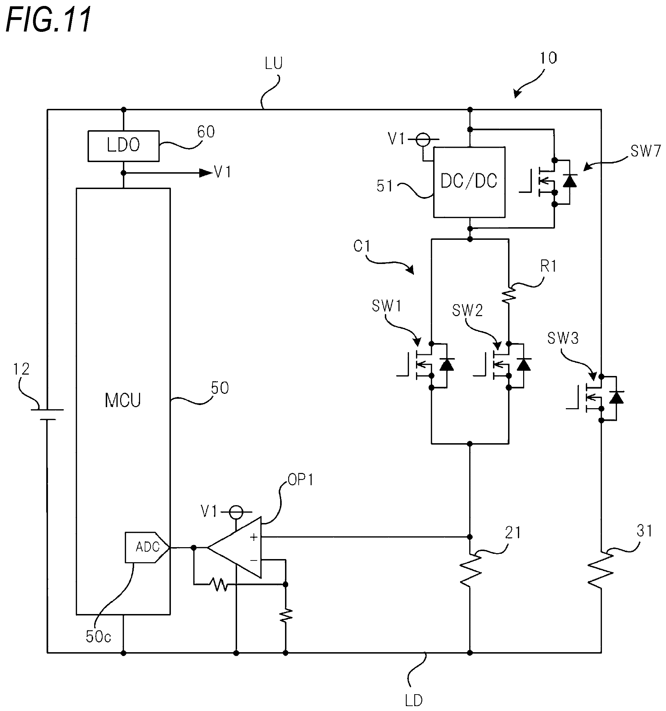

As shown in Fig. 11, instead of controlling the boosting stop of the DC/DC converter 51 described above, in the circuit shown in Fig. 8, a configuration in which a bypass circuit connected in parallel to the DC/DC converter 51 (a switch SW7) is added may be adopted. In this configuration, when boosting by the DC/DC converter 51 is unnecessary, the MCU 50 controls the switch SW7 to be in a conductive state, and causes discharging to be performed from the power supply 12 to the first load 21 via the switch SW7 without passing through the DC/DC converter 51. Generally, since the switch SW7 has a resistance value lower than that of the DC/DC converter 51 in which boosting is stopped, a power loss due to conduction can be reduced by passing through the switch SW7 in this way. Further, when boosting by the DC/DC converter 51 is required, the MCU 50 controls the switch SW7 to be in an interruption state, and causes a voltage boosted by the DC/DC converter 51 to be discharged to the first load 21. Accordingly, compared with a case where stop control of the DC/DC converter 51 is performed, the discharging control of the first load 21 can be simplified and a cost of the MCU 50 can be reduced. Further, a power loss when boosting is unnecessary can also be reduced.

After starting heating of the first load 21 in Step S19, the MCU 50 continues heating when the aerosol generation request is not ended (Step S20: NO), and stops the power supply to the first load 21 when the aerosol generation request is ended (Step S20: YES) (Step S21).

In Step S15, when the temperature $T_{cap\_sense}$ is equal to or higher than the target temperature $T_{cap\_target}$ (Step S15: YES), the MCU 50 supplies the atomization power $P_{liquid}$ (first power) determined in Step S5 to the first load 21 to start heating the first load 21 to generate an aerosol (Step S17).

After starting the heating of the first load 21 in Step S17, the MCU 50 continues heating when the aerosol generation request is not ended (Step S18: NO), and stops the power supply to the first load 21 when the aerosol generation request is ended (Step S18: YES) (Step S21).

The MCU 50 may control the heating of the first load 21 in Steps S17 and S19 based on an output of the temperature detection element T2. For example, if the MCU 50 executes the PID control or the ON/OFF control with a boiling point of the aerosol source 22 set as the target temperature based on the output of the temperature detection element T2, overheating of the first load 21 and the aerosol source 22 can be prevented, and an amount of the aerosol source 22 atomized by the first load 21 can be highly controlled.

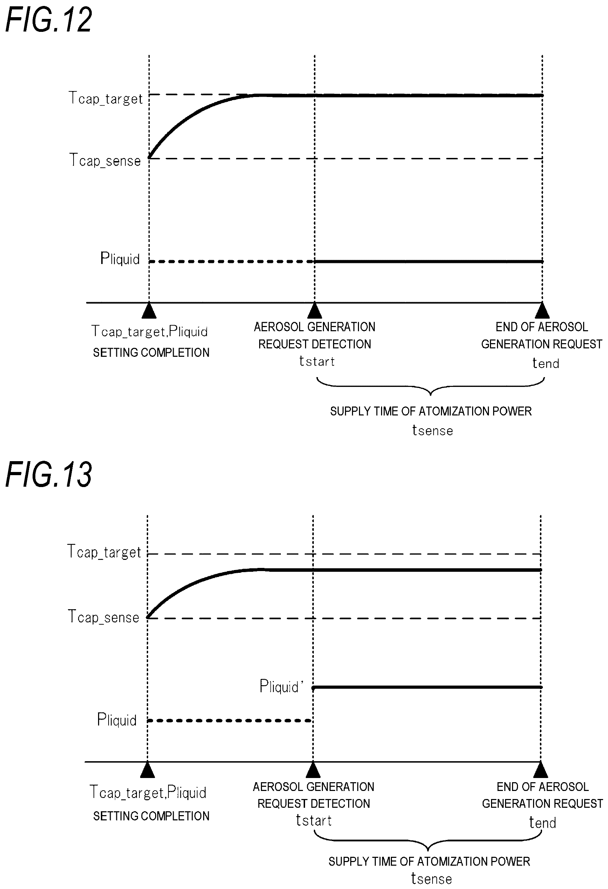

Fig. 12 is a schematic diagram showing the atomization power supplied to the first load 21 in Step S17 of Fig. 10. Fig. 13 is a schematic diagram showing the atomization power supplied to the first load 21 in Step S19 of Fig. 10. As shown in Fig. 13, when the temperature $T_{cap\_sense}$ does not reach the target temperature $T_{cap\_target}$ at a time point at which the aerosol generation request is detected, the atomization power $P_{liquid}$ is increased and then the increased atomization power $P_{liquid}$ is supplied to the first load 21.

Accordingly, even when the temperature of the flavor source 33 does not reach the target temperature at a time point at which the aerosol generation request is made, by performing the processing of Step S19, an amount of a generated aerosol can be increased. As a result, a decrease in an amount of a flavor component added to an aerosol due to the temperature of the flavor source 33 being lower than the target temperature can be compensated for by an increase in an amount of the aerosol. Therefore, the amount of the flavor component added to the aerosol can converge to the target amount.

On the other hand, when the temperature of the flavor source 33 reaches the target temperature at the time point at which the aerosol generation request is made, a desired amount of an aerosol required to achieve the target amount of the flavor component is generated by the atomization power determined in Step S5. Therefore, the amount of the flavor component added to the aerosol can converge to the target amount.

Next, the MCU 50 acquires the supply time tsense of the atomization power, which is supplied to the first load 21 in Step S17 or Step S19, to the first load 21 (Step S22). It is noted that the supply time $t_{sense}$ is equal to the first default value $t_{upper}$ when the MCU 50 detects the aerosol generation request by exceeding the first default value $t_{upper}$. Further, the MCU 50 advances the puff number counter by "1" (Step S23).

The MCU 50 updates the flavor component remaining amount $W_{capsule}$ ($n_{puff}$) of the flavor source 33 based on the supply time $t_{sense}$ acquired in Step S22, the atomization power supplied to the first load 21 upon receiving the aerosol generation request, and the target temperature $T_{cap\_target}$ at a time point at which the aerosol generation request is detected (Step S24).

When control shown in Fig. 12 is performed, the amount of the flavor component added to the aerosol generated from a start to an end of the aerosol generation request can be obtained by the following Equation (7). ($t_{end}$ - $t_{start}$) in Equation (7) indicates the supply time $t_{sense}$.

$$W_{flavor} = \beta \times (W_{capsule}\,(n_{puff}) \times T_{cap\_target}) \times \gamma \times \alpha \times P_{liquid} \times (t_{end} - t_{start})\ (7)$$

When control shown in Fig. 13 is performed, the amount of the flavor component added to the aerosol generated from a start to an end of the aerosol generation request can be obtained by the following Equation (8). ($t_{end} - t_{start}$) in Equation (8) indicates the supply time $t_{sense}$.

$$W_{flavor} = \beta \times (W_{capsule}\,(n_{puff}) \times T_{cap\_target}) \times \gamma \times \alpha \times P_{liquid}{}' \times (t_{end} - t_{start})\ (8)$$

Accordingly, the obtained $W_{flavor}$ for each aerosol generation request is accumulated in the memory 50a, and a value of a $W_{flavor}$ at the time of generating an aerosol at this time and a value of a past $W_{flavor}$ including a $W_{flavor}$ at the time of generating an aerosol before a previous time are substituted into Equation (3), so that the flavor component remaining amount $W_{capsule}\,(n_{puff})$ after the aerosol is generated can be derived with high accuracy and updated.

After Step S24, the MCU 50 determines whether the updated flavor component remaining amount $W_{capsule}$ ($n_{puff}$) is less than a remaining amount threshold (Step S25). When the updated flavor component remaining amount $W_{capsule}\,(n_{puff})$ is equal to or larger than the remaining amount threshold (Step S25: NO), the MCU 50 shifts the processing to Step S29. When the updated flavor component remaining amount $W_{capsule}\,(n_{puff})$ is less than the remaining amount threshold (Step S25: YES), the MCU 50 causes the notification unit 45 to give a notification prompting replacement of the second cartridge 30 (Step S26). Then, the MCU 50 resets the puff number counter to an initial value (= 0), erases the value of the past $W_{flavor}$ described above, and further initializes the target temperature $T_{cap\_target}$ (Step S27).

The initialization of the target temperature $T_{cap\_target}$ means excluding a target temperature $T_{cap\_target}$ stored in the memory 50a at that time point from a set value. Therefore, even when the target temperature $T_{cap\_target}$ is initialized, the previously set target temperature $T_{cap\_target}$ remains stored in the memory 50a. The stored target temperature $T_{cap\_target}$ is used as the capsule temperature parameter $T_{capsule}$ acquired the next time the MCU 50 executes Step S2.

As another example, when Steps S1 and S2 are omitted and Step S3 is always executed, the initialization of the target temperature $T_{cap\_target}$ means setting a target temperature $T_{cap\_target}$ stored in the memory 50a at that time point to a normal temperature or a room temperature.

After Step S27, the MCU 50 returns the processing to Step S1 if the power supply is not turned off (Step S28: NO), and ends the processing when the power supply is turned off (Step S28: YES).

Here, details of the remaining amount threshold used in the determination in Step S25 will be described.

The flavor component remaining amount $W_{capsule}\,(n_{puff})$ can be expressed by the following Equation (9) by Equations (1) and (2).

[Formula 4]

$$W_{capsule}\left(n_{puff}\right) = \frac{W_{flavor}}{\beta \cdot T_{capsule} \cdot \gamma \cdot W_{aerosol}} = \frac{W_{flavor}}{\beta \cdot T_{capsule} \cdot \gamma \cdot \alpha \cdot P_{liquid} \cdot t_{sense}} \cdots \quad (9)$$

In order to implement the target amount of the flavor component $W_{flavor}$, a relationship of Equation (9) must be established under a strictest condition (a state where discharging to the first load 21 is maximally continued, the temperature of the flavor source 33 reaches an upper limit, and the voltage of the power supply 12 is at a dischargeable lowest value (an end-of-discharging voltage $V_{EOD}$)). In other words, under the strictest condition, if a left side of Equation (9) is less than a right side, the target amount of the flavor component $W_{flavor}$ cannot be implemented.

In Equation (9), since the amount of the flavor component $W_{flavor}$ is intended to converge to the target amount, the amount of the flavor component $W_{flavor}$ can be handled as a known value. In Equation (9), $\alpha$, $\beta$, and $\gamma$ are constants. In Equation (9), since the $t_{sense}$ has the first default value $t_{upper}$ as an upper limit value, the upper limit value can be substituted as a value of the strictest condition. In Equation (9), in the $T_{capsule}$, an upper limit temperature $T_{max}$ of the flavor source 33 that can be heated by the second load 31 can be substituted as the value of the strictest condition. The upper limit temperature $T_{max}$ is determined by a heat-resistant temperature of a material of a container that houses the flavor source 33 and the like. As a specific example, the upper limit temperature $T_{max}$ may be 80°C. Further, in Equation (9), in the $P_{liquid}$, the second default value $P_{upper}$ obtained by substituting the end-of-discharging voltage $V_{EOD}$ into the voltage $V_{LIB}$ in Equation (5) can be substituted as the value of the strictest condition. When these values are substituted into Equation (9), Equation (10) is obtained.

[Formula 5]

$$W_{capsule}(n_{puff})$$

$$= \frac{W_{flavor}}{\alpha \times \beta \times \gamma \times \left\{ MIN \left( \frac{(\eta_{upper} \cdot V_{EOD})^2}{R_{HTR}(T_{HTR} = T_{B.P.})} \quad P_{DC/DC\_upper} \right) - \Delta \right\} \times t_{upper} \times T_{max}} \quad \dots \quad (10)$$

Therefore, by setting the remaining amount threshold to a value on a right side of Equation (10), it is possible to prompt the user to replace the second cartridge 30 at an appropriate timing. A state where the flavor component remaining amount $W_{capsule}$ ($n_{puff}$) is less than the right side of Equation (10) constitutes any of a state where the amount of the flavor component is smaller than the target amount when the first load 21 is discharged in response to the aerosol generation request, a state where the amount of the flavor component is smaller than the target amount when the first load 21 is discharged for a maximum time (first default time $t_{upper}$) in response to the aerosol generation request, and a state where the amount of the flavor component is smaller than the target amount when dischargeable maximum power ($P_{upper}$) is supplied from the power supply 12 to the first load 21 in response to the aerosol generation request. The maximum power is power that can be supplied from the power supply 12 to the first load 21, or power dischargeable from the power supply 12 to the first load 21 in an end-of-discharging state, when a voltage of the power supply 12 is boosted to a maximum voltage that the DC/DC converter 51 can boost.

The remaining amount threshold is set in this way, so that it is possible to prompt the user to replace the second cartridge 30 in a state before the amount of the flavor component is smaller than the target amount. Therefore, the user can be prevented from sucking an aerosol to which a small amount of a flavor component that does not reach a target is added, and a commercial value of the aerosol inhaler 1 can be further increased.

(Effects of Embodiment)

As described above, according to the aerosol inhaler 1, every time the user sucks the aerosol, the discharging control from the power supply 12 to the first load 21 and the second load 31 is performed such that the amount of the flavor component contained in the aerosol converges to the target amount. Therefore, the amount of the flavor component provided to the user can be stabilized for each suction, and the commercial value of the aerosol inhaler 1 can be increased. Further, compared with a case where only the first load 21 is discharged, the amount of the flavor component for each suction provided to the user can be stabilized, and the commercial value of the aerosol inhaler 1 can be further increased.

According to the aerosol inhaler 1, when the atomization power determined in step S5 is larger than the second default value and the aerosol required to achieve the target amount of the flavor component cannot be generated, discharging from the power supply 12 to the second load 31 is controlled. Accordingly, since discharging to the second load 31 is performed as needed, the amount of the flavor component for each suction provided to the user can be stabilized and electric energy for implementing the stabilization can be reduced.

When the aerosol generation request is repeated, the voltage of the power supply 12 decreases. However, according to the aerosol inhaler 1, the target temperature is increased in response to the decrease in the voltage of the power supply 12, an amount of discharging to the second load 31 is increased, and the temperature of the flavor source 33 is controlled to converge to the target temperature. Therefore, a decrease in the amount of the flavor component due to a decrease in an amount of an aerosol due to the decrease in the voltage of the power supply 12 can be compensated for by an increase in the temperature of the flavor source 33, and the amount of the flavor component provided to the user can be stabilized.

According to the aerosol inhaler 1, based on a time of discharging to the first load 21 ($t_{sense}$) in response to the aerosol generation request, the $T_{cap\_target}$ at a time point at which the generation request is received, and the power (the atomization power $P_{liquid}$, the atomization power $P_{liquid}'$) or electric energy (the power $\times$ $t_{sense}$)) discharged to the first load in response to the generation request, the flavor component remaining amount is updated in Step S24. Based on the flavor component remaining amount, the power to be discharged to the first load 21 is determined in Steps S4 and S5. Therefore, the power or the electric energy discharged to the first load 21 that has a great influence on the amount of the flavor component that can be added to the aerosol is appropriately considered, the temperature of the flavor source 33 when discharging to the first load 21 that has a great influence on the amount of the flavor component that can be added to the aerosol is appropriately considered, and then discharging to the first load 21 can be controlled. Accordingly, by controlling the discharging to the first load 21 after appropriately considering a state of the aerosol inhaler 1, the amount of the flavor component for each suction can be stabilized with high accuracy, and the commercial value of the aerosol inhaler 1 can be increased.

According to the aerosol inhaler 1, the flavor source 33 is heated before the aerosol generation request is detected. Therefore, the flavor source 33 can be warmed before aerosol generation, and time required from receiving the aerosol generation request to generating an aerosol to which a desired amount of a flavor component is added can be shortened.

According to the aerosol inhaler 1, discharging to the second load 31 is stopped after receiving the aerosol generation request. Therefore, the first load 21 and the second load 31 are not discharged at the same time, and shortage of power discharged to the second load 31 can be prevented. In addition, discharging of a large current from the power supply 12 is prevented. Therefore, deterioration of the power supply 12 can be prevented.

According to the aerosol inhaler 1, by resuming discharging to the second load 31 after an aerosol is generated, a state where the flavor source 33 is warmed can be maintained even when the aerosol is continuously generated. Therefore, it is possible to provide the user with a stable amount of a flavor component over a plurality of consecutive suctions.

(First Modification of Aerosol Inhaler)

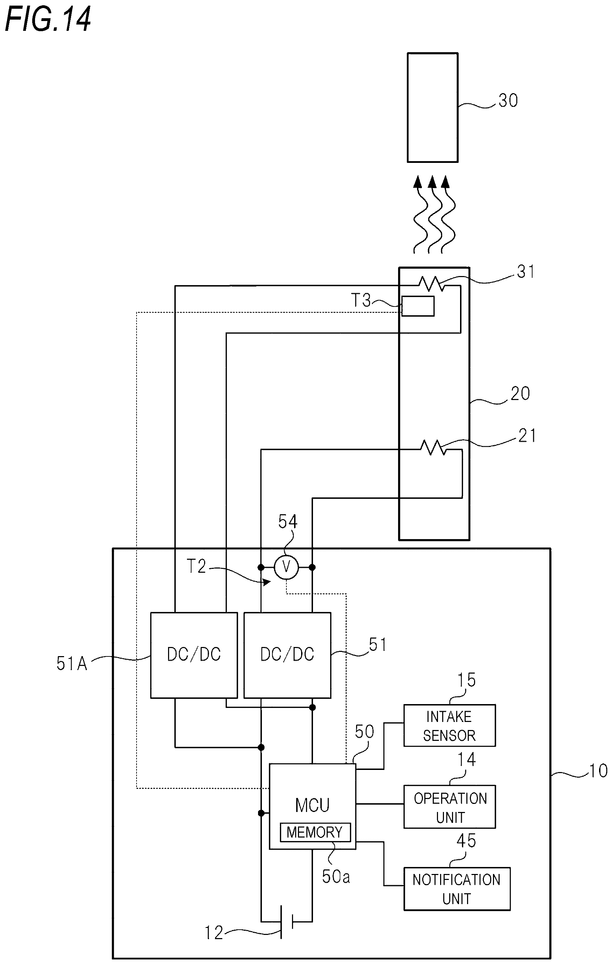

Fig. 14 is a schematic diagram showing a first modification of the hardware configuration of the aerosol inhaler of Fig. 1. Fig. 14 shows a configuration obtained by eliminating the current sensor 55 from the configuration of Fig. 6 and adding a DC/DC converter 51A as a boosting circuit to the configuration of Fig. 6.

The DC/DC converter 51A is connected to a connection node between the DC/DC converter 51 and the MCU 50 and the second load 31. That is, in the power supply unit 10 shown in Fig. 14, in a state where the first cartridge 20 is mounted, the series circuit of the DC/DC converter 51 and the first load 21 and a series circuit of the DC/DC converter 51A and the second load 31 are connected in parallel to the power supply 12.

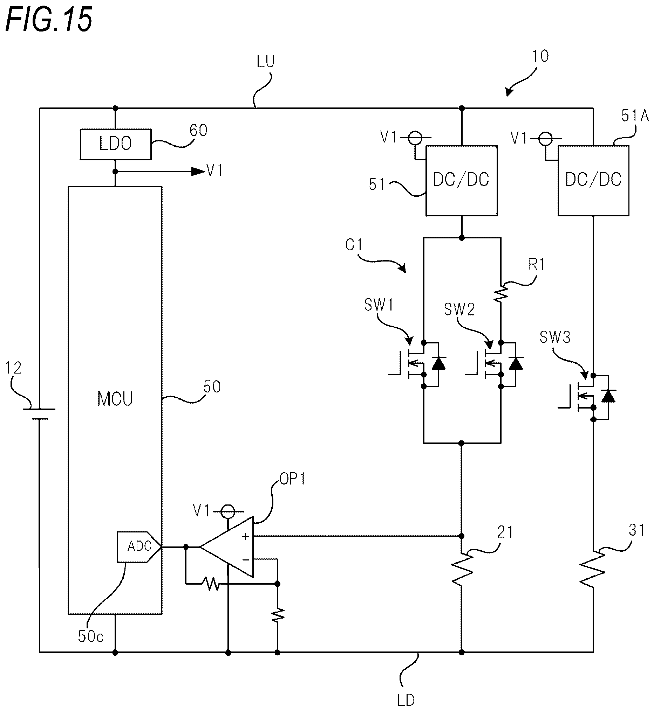

Fig. 15 is a diagram showing a specific example of the power supply unit 10 shown in Fig. 14. A circuit shown in Fig. 15 has the same configuration as that of Fig. 8 except that the DC/DC converter 51A is added between the main positive bus LU and the switch SW3. Specifically, an input terminal of the DC/DC converter 51A is connected to the main positive bus LU, and an output terminal of the DC/DC converter 51A is connected to the switch SW3.

According to the first modification, the DC/DC converter 51A can appropriately control a voltage applied to the second load 31 and can apply a voltage different from that of the first load 21 to the second load 31. As a result, an amount of a flavor component added to an aerosol can be controlled more flexibly.

(Second Modification of Aerosol Inhaler)

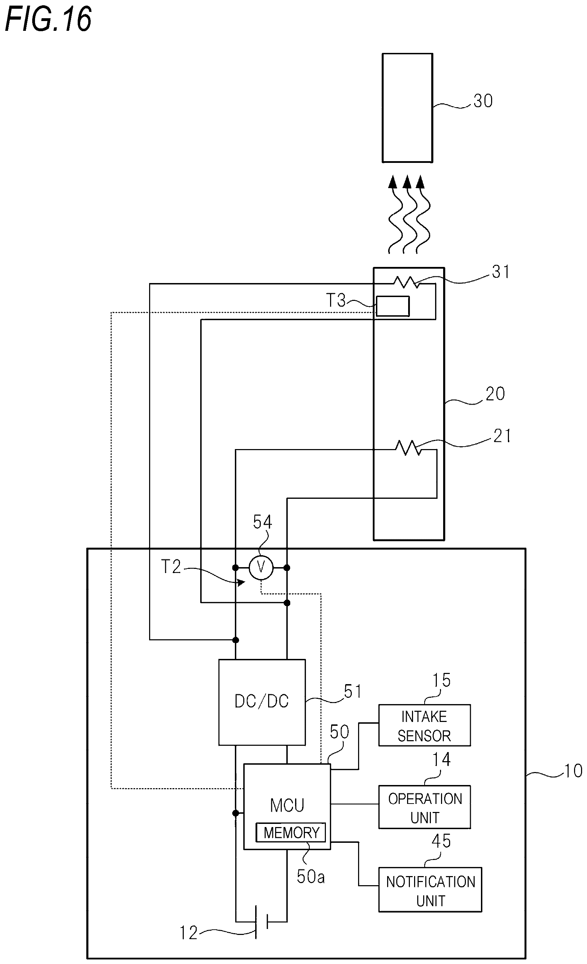

Fig. 16 is a schematic diagram showing a second modification of the hardware configuration of the aerosol inhaler of Fig. 1. Fig. 16 shows a configuration obtained by eliminating the DC/DC converter 51A from the configuration shown in Fig. 14 and also connecting an output of the DC/DC converter 51 to the second load 31 in the configuration shown in Fig. 14. That is, in the power supply unit 10 shown in Fig. 16, the first load 21 and the second load 31 are connected in parallel to the DC/DC converter 51.

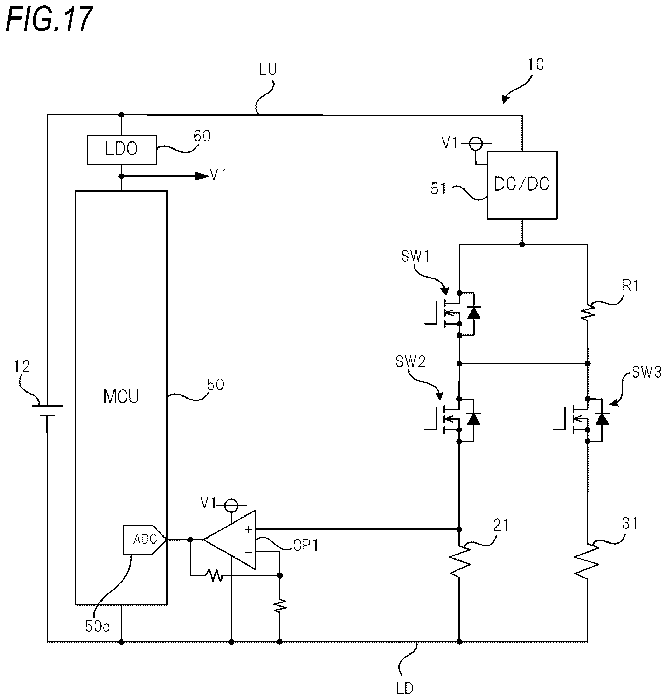

Fig. 17 is a diagram showing a specific example of the power supply unit 10 shown in Fig. 16. A circuit shown in Fig. 17 has a configuration in which connection positions between the switches SW1 to SW3 and the resistance element R1 are changed in the circuit shown in Fig. 8. In the circuit shown in Fig. 17, the switch SW2 is connected to a terminal on a high potential side of the first load 21, and the switch SW1 is connected to the switch SW2 and an output terminal of the DC/DC converter 51. Further, the switch SW3 is connected to a terminal on a high potential side of the second load 31, and the resistance element R1 is connected to the switch SW3 and an output terminal of the DC/DC converter 51. Further, a connection node between the switch SW1 and the switch SW2 is connected to a connection node between the resistance element R1 and the switch SW3.

In a circuit configuration shown in Fig. 17, a temperature detection unit of the MCU 50 acquires an output value of the ADC 50c (a value of a voltage applied to the first load 21) and acquires a temperature of the first load 21 based on the output value, in a state where the switch SW1 and the switch SW3 are controlled to be in an interruption state and the switch SW2 is controlled to be in a conductive state. Further, a power control unit of the MCU 50 controls the switch SW3 to be in an interruption state and controls the switch SW1 and the switch SW2 to be in a conductive state, so that discharging is performed from the power supply 12 to the first load 21 to atomize the aerosol source 22. Further, the power control unit controls the switch SW2 to be in an interruption state and controls the switch SW1 and the switch SW3 to be in a conductive state, so that discharging is performed from the power supply 12 to the second load 31 to heat the flavor source 33.

According to the second modification, the DC/DC converter 51 can appropriately control a voltage applied to the first load 21 and the second load 31. As a result, an amount of a flavor component added to an aerosol can be controlled more flexibly. Further, compared with the first modification, the DC/DC converter 51A can be omitted, so that a circuit scale can be suppressed.

In the circuit shown in Fig. 17, in a state where the switch SW1 and the switch SW3 are controlled to be in an interruption state and the switch SW2 is controlled to be in a conductive state, the circuit configuration may be changed such that the output value of the ADC 50c is a value of a voltage applied to the resistance element R1, and the temperature detection unit of the MCU 50 may acquire the temperature of the first load 21 based on the output value.

(Third Modification of Aerosol Inhaler)

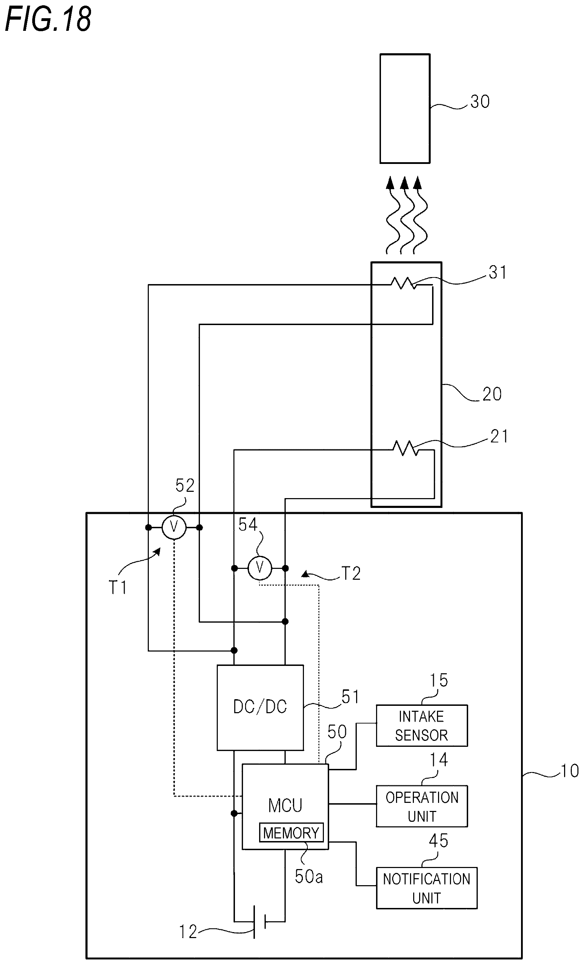

Fig. 18 is a schematic diagram showing a third modification of the hardware configuration of the aerosol inhaler of Fig. 1. Fig. 18 shows a configuration obtained by adding the voltage sensor 52 that constitutes the temperature detection element T1 to the configuration shown in Fig. 16, instead of using the temperature detection element T3.

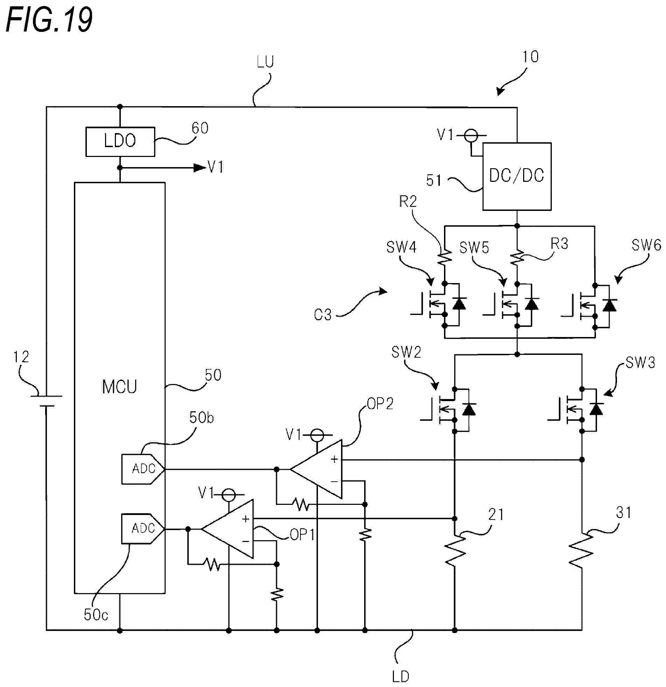

Fig. 19 is a diagram showing a specific example of the power supply unit 10 shown in Fig. 18. A circuit shown in Fig. 19 has a configuration obtained by adding the operational amplifier OP2 and the ADC 50b that constitute the voltage sensor 52 to the circuit shown in Fig. 17, eliminating the resistance element R1 and the switch SW1 from the circuit shown in Fig. 17, and adding the switches SW4 to SW6, the resistance element R2, and a resistance element R3 to the circuit shown in Fig. 17.

In the circuit shown in Fig. 19, a non-inverting input terminal of the operational amplifier OP2 is connected to a connection node between the second load 31 and the switch SW3. An inverting input terminal of the operational amplifier OP2 is connected to an output terminal of the operational amplifier OP2 and the main negative bus LD via a resistance element. Further, a parallel circuit C3 is connected to a connection node between the switch SW2 and the switch SW3 and an output of the DC/DC converter 51.

In the parallel circuit C3, a series circuit of the resistance element R2 and the switch SW4, a series circuit of the resistance element R3 and the switch SW5, and the switch SW6 are connected in parallel. A terminal on a high potential side of the resistance element R2 is connected to an output terminal of the DC/DC converter 51. A terminal on a low potential side of the switch SW4 is connected to the connection node between the switch SW2 and the switch SW3. A terminal on a high potential side of the resistance element R3 is connected to the output terminal of the DC/DC converter 51. A terminal on a low potential side of the switch SW5 is connected to the connection node between the switch SW2 and the switch SW3. A terminal on a high potential side of the switch SW6 is connected to the output terminal of the DC/DC converter 51, and a terminal on a low potential side of the switch SW6 is connected to the connection node between the switch SW2 and the switch SW3.

In a circuit configuration shown in Fig. 19, a temperature detection unit of the MCU 50 acquires an output value of the ADC 50c (a value of a voltage applied to the first load 21) and acquires a temperature of the first load 21 based on the output value, in a state where the switch SW3, the switch SW5, and the switch SW6 are controlled to be in an interruption state and the switch SW2 and the switch SW4 are controlled to be in a conductive state. Further, the temperature detection unit of the MCU 50 acquires an output value of the ADC 50b (a value of a voltage applied to the second load 31) and acquires a temperature of the second load 31 based on the output value, in a state where the switch SW2, the switch SW4, and the switch SW6 are controlled to be in an interruption state and the switch SW3 and the switch SW5 are controlled to be in a conductive state.

A power control unit of the MCU 50 controls the switch SW3, the switch SW4, and the switch SW5 to be in an interruption state and controls the switch SW2 and the switch SW6 to be in a conductive state, so that discharging is performed from the power supply 12 to the first load 21 to atomize the aerosol source 22. The power control unit of the MCU 50 controls the switch SW2, the switch SW4, and the switch SW5 to be in an interruption state and controls the switch SW3 and the switch SW6 to be in a conductive state, so that discharging is performed from the power supply 12 to the second load 31 to heat the flavor source 33.

According to the third modification, the temperature of the second load 31 can be acquired without providing the temperature detection element T3 on the second cartridge 30. Therefore, a state of the flavor source 33 can be grasped with an inexpensive configuration. Further, since the temperature of the first load 21 can also be acquired without providing a dedicated sensor on the second cartridge 30, a state of the aerosol source 22 can be grasped with an inexpensive configuration. Further, since the resistance element R2 used to acquire the temperature of the first load 21 and the resistance element R3 used to acquire the temperature of the second load 31 are provided separately, the optimal resistance element R2 and the optimal resistance element R3 can be used according to performance and specifications of the operational amplifier OP1, the operational amplifier OP2, the ADC50b, and the ADC 50c.

In the circuit shown in Fig. 19, in a state where the switch SW3, the switch SW5, and the switch SW6 are controlled to be in an interruption state and the switch SW2 and the switch SW4 are controlled to be in a conductive state, the circuit configuration may be changed such that the output value of the ADC 50c is a value of a voltage applied to the resistance element R2, and the temperature detection unit of the MCU 50 may acquire the temperature of the first load 21 based on the output value.

Similarly, in the circuit shown in Fig. 19, in a state where the switch SW2, the switch SW4, and the switch SW6 are controlled to be in an interruption state and the switch SW3 and the switch SW5 are controlled to be in a conductive state, the circuit configuration may be changed such that the output value of the ADC 50b is a value of a voltage applied to the resistance element R3, and the temperature detection unit of the MCU 50 may acquire the temperature of the second load 31 based on the output value.

(Fourth Modification of Aerosol Inhaler)

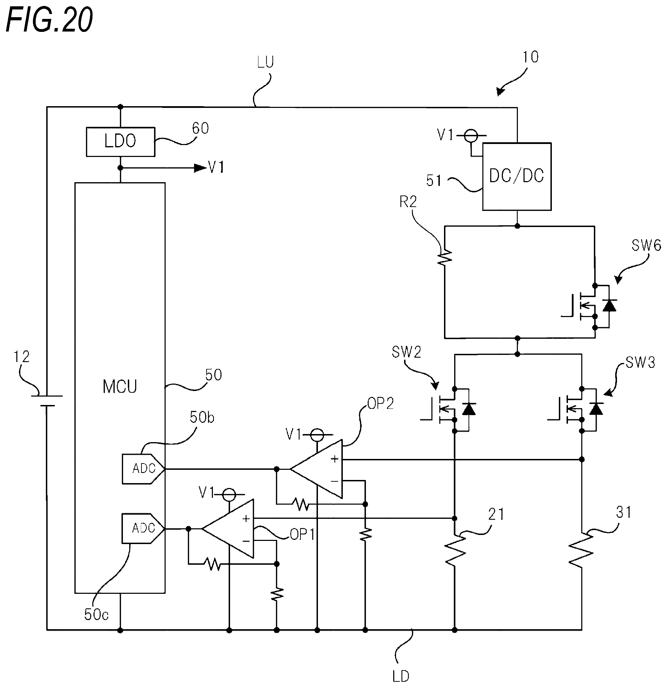

Fig. 20 is a diagram showing a modification of the specific example of the power supply unit 10 shown in Fig. 18. A circuit shown in Fig. 20 has a configuration obtained by eliminating the resistance element R3, the switch SW4, and the switch SW5 from the circuit shown in Fig. 19.

In a circuit configuration shown in Fig. 20, a temperature detection unit of the MCU 50 acquires an output value of the ADC 50c (a value of a voltage applied to the first load 21) and acquires a temperature of the first load 21 based on the output value, in a state where the switch SW3 and the switch SW6 are controlled to be in an interruption state and the switch SW2 is controlled to be in a conductive state. Further, a power control unit of the MCU 50 acquires an output value of the ADC 50b (a value of a voltage applied to the second load 31) and acquires a temperature of the second load 31 based on the output value, in a state where the switch SW2 and the switch SW6 are controlled to be in an interruption state and the switch SW3 is controlled to be in a conductive state.

The power control unit of the MCU 50 controls the switch SW3 to be in an interruption state and controls the switch SW2 and the switch SW6 to be in a conductive state, so that discharging is performed from the power supply 12 to the first load 21 to atomize the aerosol source 22. Further, the power control unit of the MCU 50 controls the switch SW2 to be in an interruption state and controls the switch SW3 and the switch SW6 to be in a conductive state, so that discharging is performed from the power supply 12 to the second load 31 to heat the flavor source 33.

According to the fourth modification, only one resistance element R2 is provided, and the temperatures of the first load 21 and the second load 31 can be acquired from the sensors in the circuit without disposing a dedicated temperature detection element in the vicinity of the second load 31. Therefore, states of the aerosol source 22 and the flavor source 33 can be grasped with a more inexpensive configuration. Further, compared with the third modification, a power loss of the parallel circuit connected to the output of the DC/DC converter 51 can be prevented. Therefore, discharging for generating an aerosol to which a flavor component is added and discharging for acquiring the temperatures of the first load 21 and the second load 31 can be performed with low power consumption.

In the circuit shown in Fig. 20, in a state where the switch SW3 and the switch SW6 are controlled to be in an interruption state and the switch SW2 is controlled to be in a conductive state, the circuit configuration may be changed such that an output value of the ADC 50c is a value of a voltage applied to the resistance element R2, and the temperature detection unit of the MCU 50 may acquire the temperature of the first load 21 based on the output value.

Similarly, in the circuit shown in Fig. 20, in a state where the switch SW2 and the switch SW6 are controlled to be in an interruption state and the switch SW3 is controlled to be in a conductive state, the circuit configuration may be changed such that an output value of the ADC 50b is the value of the voltage applied to the resistance element R2, and the temperature detection unit of the MCU 50 may acquire the temperature of the second load 31 based on the output value.

(Fifth Modification of Aerosol Inhaler)

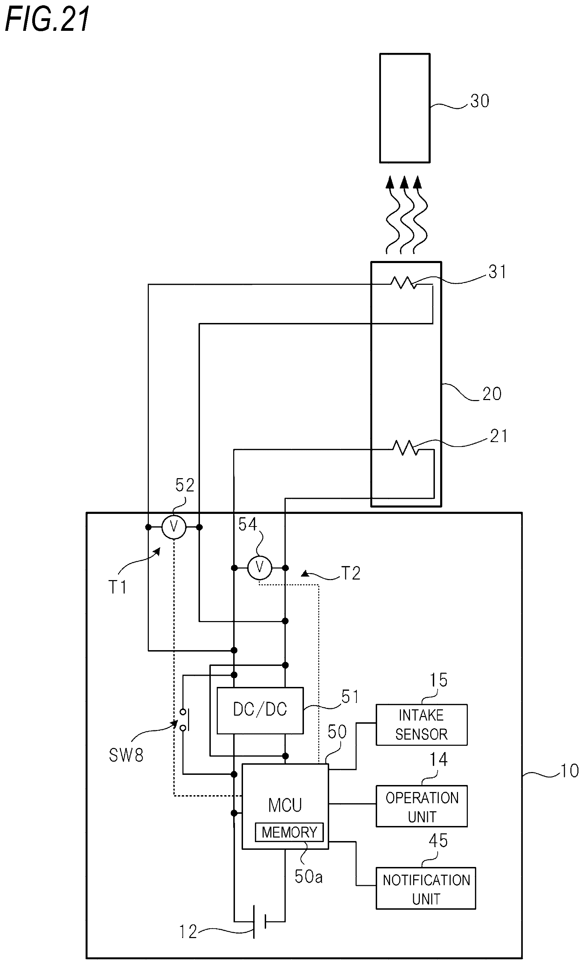

Fig. 21 is a schematic diagram showing a fifth modification of the hardware configuration of the aerosol inhaler of Fig. 1. Fig. 21 shows a configuration obtained by adding a switch SW8 connected in parallel to the DC/DC converter 51 to the configuration shown in Fig. 18.

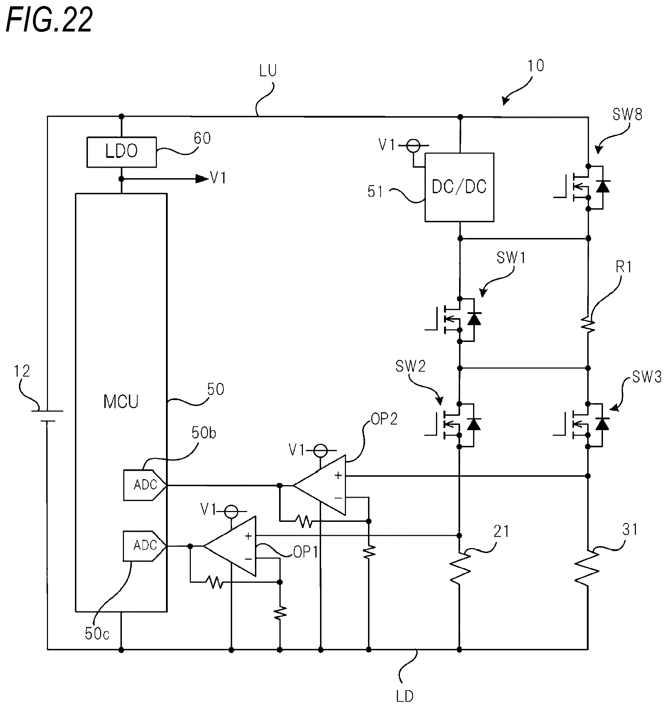

Fig. 22 is a diagram showing a specific example of the power supply unit 10 shown in Fig. 21. A circuit shown in Fig. 22 has a configuration obtained by adding the switch SW8, and the operational amplifier OP2 and the ADC 50b that constitute the voltage sensor 52 to the circuit shown in Fig. 17.

In the circuit shown in Fig. 22, a non-inverting input terminal of the operational amplifier OP2 is connected to a connection node between the second load 31 and the switch SW3. An inverting input terminal of the operational amplifier OP2 is connected to an output terminal of the operational amplifier OP2 and the main negative bus LD via a resistance element. The switch SW8 is connected to a terminal on a high potential side of the resistance element R1 and the main positive bus LU. An output terminal of the DC/DC converter 51 is connected to the switch SW1. A connection node between the DC/DC converter 51 and the switch SW1 is connected to a connection node between the switch SW8 and the resistance element R1.

In the circuit configuration shown in Fig. 22, in a state where the switch SW1 and the switch SW3 are controlled to be in an interruption state and the switch SW2 and the switch SW8 are controlled to be in a conductive state, a temperature detection unit of the MCU 50 acquires an output value of the ADC 50c (a value of a voltage applied to the first load 21) and acquires a temperature of the first load 21 based on the output value. Further, in a state where the switch SW1 and the switch SW2 are controlled to be in an interruption state and the switch SW3 and the switch SW8 are controlled to be in a conductive state, the temperature detection unit of the MCU 50 acquires an output value of the ADC 50b (a value of a voltage applied to the second load 31) and acquires a temperature of the second load 31 based on the output value.

A power control unit of the MCU 50 controls the switch SW3 to be in an interruption state, controls the switch SW1 and the switch SW2 to be in a conductive state, and controls the switch SW8 to be in an interruption state, so that a voltage boosted by the DC/DC converter 51 is discharged to the first load 21. At the same time, the output value of the ADC 50c (the value of the voltage applied to the first load 21) may be acquired, and the temperature of the first load 21 may be acquired based on the output value. The power control unit of the MCU 50 controls the switch SW3 to be in an interruption state, controls the switch SW1 and the switch SW2 to be in a conductive state, and controls the switch SW8 to be in a conductive state, so that a voltage from the power supply 12 is discharged to the first load 21 without being boosted by the DC/DC converter 51.

The power control unit controls the switch SW2 to be in an interruption state, controls the switch SW1 and the switch SW3 to be in a conductive state, and controls the switch SW8 to be in an interruption state, so that a voltage boosted by the DC/DC converter 51 is discharged to the second load 31. At the same time, the output value of the ADC 50b (the value of the voltage applied to the second load 31) may be acquired, and the temperature of the second load 31 may be acquired based on the output value. The power control unit of the MCU 50 controls the switch SW2 to be in an interruption state, controls the switch SW1 and the switch SW3 to be in a conductive state, and controls the switch SW8 to be in a conductive state, so that the voltage from the power supply 12 is discharged to the second load 31 without being boosted by the DC/DC converter 51.

According to the fifth modification, the switch SW8 can supply the voltage from the power supply 12 to the load without passing through the DC/DC converter 51. Therefore, when boosting is unnecessary, discharging to the load can be performed with higher efficiency.

In the circuit shown in Fig. 22, in a state where the switch SW1 and the switch SW3 are controlled to be in an interruption state and the switch SW2 and the switch SW8 are controlled to be in a conductive state, the circuit configuration may be changed such that the output value of the ADC 50c is a value of a voltage applied to the resistance element R1, and the temperature detection unit of the MCU 50 may acquire the temperature of the first load 21 based on the output value.

Similarly, in the circuit shown in Fig. 22, in a state where the switch SW1 and the switch SW2 are controlled to be in an interruption state and the switch SW3 and the switch SW8 are controlled to be in a conductive state, the circuit configuration may be changed such that the output value of the ADC 50b is the value of the voltage applied to the resistance element R1, and the temperature detection unit of the MCU 50 may acquire the temperature of the second load 31 based on the output value.

(Sixth Modification of Aerosol Inhaler)

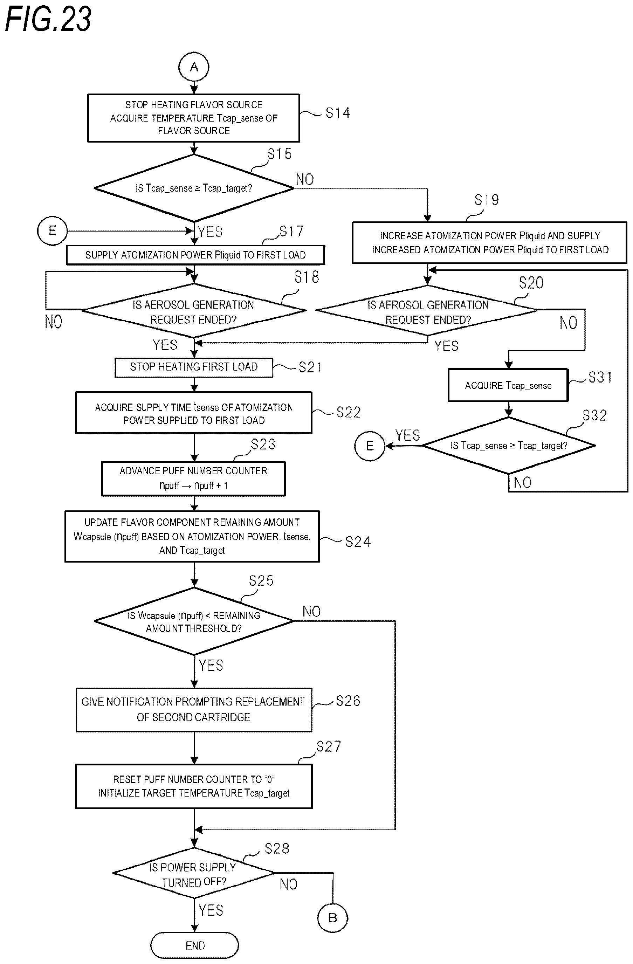

Fig. 23 is a flowchart for illustrating a modification of the operations of the aerosol inhaler 1 of Fig. 1. Fig. 23 shows a modification of Step S14 and steps after Step S14 in the flowcharts shown in Figs. 9 and 10. The flowchart shown in Fig. 23 is different from that of Fig. 10 in that when determination in Step S20 is NO, a processing shifts to Step S31 and Step S32 instead of returning to Step S20.

In Step S31, the MCU 50 acquires the temperature $T_{cap\_sense}$ of the flavor source 33 at that time point based on an output of the temperature detection element T1 (or the temperature detection element T3). In Step S32 after Step S31, the MCU 50 performs the same processing as that of Step S15. Then, the MCU 50 shifts the processing to Step S20 when determination in Step S32 is NO, and shifts the processing to Step S17 when the determination in Step S32 is YES.

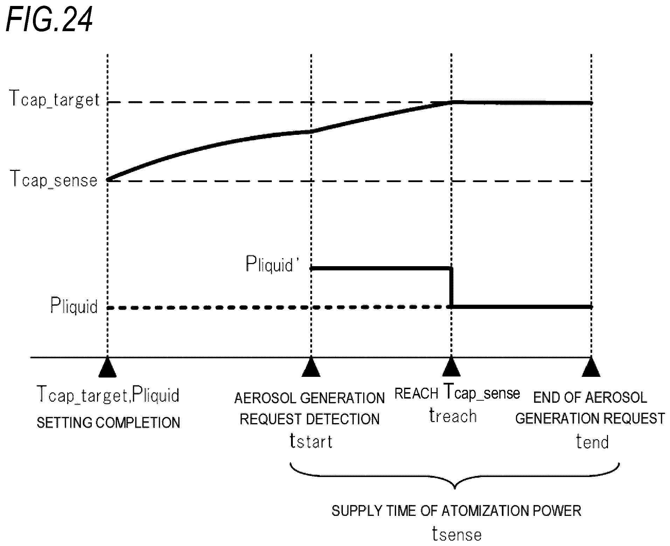

Fig. 24 is a schematic diagram showing a change in atomization power when the determination in Step S15 in Fig. 23 is NO, then the determination in Step S20 is NO, and then the determination in Step S32 is YES.

As shown in Fig. 24, when the temperature $T_{cap\_sense}$ does not reach the target temperature $T_{cap\_target}$ at a time point at which an aerosol generation request is detected, the atomization power $P_{liquid}$ is increased and then the increased atomization power $P_{liquid}$ is supplied to the first load 21. Therefore, the temperature $T_{cap\_sense}$ approaches the target temperature $T_{cap\_target}$ by supplying the increased atomization power $P_{liquid}$' to the first load 21. When the temperature $T_{cap\_sense}$ reaches the target temperature $T_{cap\_target}$ at a time $t_{reach}$ shown in Fig. 24 while the aerosol generation request is continued, the atomization power is reduced and returns to an original value (a value determined in Step S5 in Fig. 9). Then, the state is continued until the aerosol generation request is ended.

When the control shown in Fig. 24 is performed, an amount of a flavor component added to an aerosol generated from a start to an end of the aerosol generation request can be obtained by the following Equation (11). A sum of ($t_{reach}$ - $t_{start}$) and ($t_{end}$ - $t_{reach}$) in Equation (11) indicates the supply time tsense during which power is supplied to the first load 21. Accordingly, by using the obtained amount of the flavor component, it is possible to accurately update a flavor component remaining amount.

$$W_{flavor}$$

$$= \beta \times \{(W_{capsule}(n_{puff}) \times T_{cap\_target}) \times \gamma \times \alpha \times P_{liquid} \times (t_{end} - t_{reach}) + (W_{capsule}(n_{puff}) \times T_{cap\_target}) \times \gamma \times \alpha \times P_{liquid'} \times (t_{reach} - t_{start})\}$$

$$(11)$$

According to the modification, since the control shown in Fig. 24 is performed in addition to the control shown in Fig. 12 and the control shown in Fig. 13, power to be supplied to the first load 21 during the generation of the aerosol can be reduced. Therefore, power consumption can be suppressed. Further, since the amount of the flavor component added to the aerosol generated from the start to the end of the aerosol generation request is highly stable, a commercial value of the aerosol inhaler 1 can be further increased.

It is assumed that when the processing is shifted from Step S32 to Step S17 in Fig 23, atomization power to be supplied to the first load 21 (power changed by the MCU 50) is a value that can be discharged from the power supply 12 to the first load 21 without boosting by the DC/DC converter 51 (in other words, even when the boosting by the DC/DC converter 51 is stopped). In this case, it is preferable that the MCU 50 controls a switching element of the DC/DC converter 51 such that the DC/DC converter 51 outputs an input voltage as it is, and supplies a voltage from the power supply 12 to the first load 21 without boosting the voltage. Accordingly, power consumption can be suppressed by reducing a loss in association with boosting by the DC/DC converter 51.

On the other hand, it is assumed that when the processing is shifted from Step S32 to Step S17 in Fig. 23, the atomization power to be supplied to the first load 21 is a value that cannot be discharged from the power supply 12 to the first load 21 without boosting by the DC/DC converter 51. In this case, the MCU 50 may control the switching element of the DC/DC converter 51 such that the DC/DC converter 51 boosts the input voltage and outputs the boosted voltage, and boost the voltage from the power supply 12 to supply the boosted voltage to the first load 21. Accordingly, it is possible to supply required power to the first load 21 while suppressing power consumption.

Further, it is assumed that the circuit configuration shown in Fig. 11 is adopted and the processing is shifted from Step S32 to Step S17 in Fig. 22. In this case, if boosting is unnecessary, the MCU 50 controls the switch SW7 to be in a conductive state, and causes discharging to be performed from the power supply 12 to the first load 21 via the switch SW7 without passing through the DC/DC converter 51. Generally, since the switch SW7 has a resistance value lower than that of the DC/DC converter 51 in which boosting is stopped, a power loss due to conduction can be reduced by passing through the switch SW7 in this way. Further, if boosting is necessary, the MCU 50 controls the switch SW7 to be in an interruption state and causes a voltage boosted by the DC/DC converter 51 to be discharged to the first load 21. Accordingly, compared with a case where stop control of the DC/DC converter 51 is performed, discharging control of the first load 21 can be simplified and a cost of the MCU 50 can be reduced. Further, a conduction loss when boosting is unnecessary can also be reduced.

In the above embodiment and modifications, the configuration is provided in which the first cartridge 20 is detachable from the power supply unit 10, but a configuration may be provided in which the first cartridge 20 is integrated with the power supply unit 10.

In the above embodiment and modifications, the first load 21 and the second load 31 are heaters that generate heat by power discharged from the power supply 12, but the first load 21 and the second load 31 may be Peltier elements that can perform both heat generation and cooling by the power discharged from the power supply 12. If the first load 21 and the second load 31 are configured in this way, a degree of freedom of control related to the temperature of the aerosol source 22 and the temperature of the flavor source 33 is increased, so that the unit flavor amount can be controlled more highly.

Further, the first load 21 may be configured with an element that can atomize the aerosol source 22 without heating the aerosol source 22 by ultrasonic waves or the like. Further, the second load 31 may be configured with an element that can change the amount of the flavor component added to the aerosol by the flavor source 33 without heating the flavor source 33 by the ultrasonic waves or the like.

When, for example, an ultrasonic wave element is used for the second load 31, the MCU 50 may control discharging to the first load 21 and the second load 31 based on, for example, a wavelength of ultrasonic waves applied to the flavor source 33, instead of the temperature of the flavor source 33 as a parameter that influences an amount of a flavor component added to an aerosol that passes through the flavor source 33.

An element that can be used for the first load 21 is not limited to the heater, the Peltier element, and the ultrasonic wave element described above, and various elements or combinations thereof can be used as long as the element can atomize the aerosol source 22 by consuming the power supplied from the power supply 12. Similarly, an element that can be used for the second load 31 is not limited to the heater, the Peltier element, and the ultrasonic wave element described above, and various elements or combinations thereof can be used as long as the element can change the amount of the flavor component added to the aerosol by consuming the power supplied from the power supply 12.

In the above description, the MCU 50 controls discharging from the power supply 12 to the first load 21 and the second load 31 such that the amount of the flavor component $W_{flavor}$ converges to the target amount. The target amount is not limited to one specific value and may be in a range having a certain width.

In the above description, the MCU 50 controls discharging from the power supply 12 to the second load 31 such that the temperature of the flavor source 33 converges to the target temperature. The target temperature is not limited to one specific value and may be in a range having a certain width.

At least the following matters are described in the present description. Corresponding components in the above embodiment are shown in parentheses. However, the present invention is not limited thereto.

(1) A power supply unit (a power supply unit 10) for an aerosol inhaler (an aerosol inhaler 1) that causes an aerosol generated from an aerosol source (an aerosol source 22) to pass through a flavor source (a flavor source 33) to add a flavor component of the flavor source to the aerosol, the power supply unit including:

a power supply (a power supply 12) configured to be dischargeable to a first load (a first load 21) configured to heat the aerosol source; and

a processing device (an MCU 50) configured to cause determined power to be discharged from the power supply to the first load in response to a signal from a sensor (an intake sensor 15 or an operation unit 14) configured to output the signal indicating an aerosol generation request,

in which the processing device is configured to determine the power based on a time of discharging ($t_{sense}$) and a variable different from the time in the discharging from the power supply to the first load in response to the signal before a previous time.

According to (1), since the power to be discharged to the first load is determined based on the time of discharging to the first load in the past and the variable different from the time of discharging, the discharging to the first load can be controlled after appropriately considering a state of the aerosol inhaler (particularly, an amount of a flavor component that can be added to an aerosol). Therefore, accuracy of controlling the discharging to the first load for generating an aerosol to which a desired amount of a flavor component is added can be improved. As a result, the amount of the flavor component for each suction can be stabilized with high accuracy, and a commercial value of the aerosol inhaler can be increased.

(2) The power supply unit according to (1),

in which the variable is power (atomization power $P_{liquid}$, atomization power $P_{liquid}'$) or electric energy (atomization power $\times t_{sense}$) discharged from the power supply to the first load.

According to (2), the discharging to the first load is controlled after appropriately considering the power or the electric energy discharged to the first load that has a great influence on the amount of the flavor component that can be added to the aerosol. Therefore, the accuracy of controlling the discharging to the first load for generating the aerosol to which the desired amount of the flavor component is added can be further improved.

(3) The power supply unit according to (1) or (2),

in which the processing device is configured to acquire an output value ($T_{cap\_target}$) of an element (a memory 50a) configured to output information on a temperature of the flavor source and use the output value as the variable.

According to (3), the discharging to the first load is controlled after appropriately considering the temperature of the flavor source when discharging to the first load that has a great influence on the amount of the flavor component that can be added to the aerosol. Therefore, the accuracy of controlling the discharging to the first load for generating the aerosol to which the desired amount of the flavor component is added can be further improved.

(4) The power supply unit according to (3),

in which while discharging the power to the first load, the processing device is configured to acquire a temperature ($T_{cap\_sense}$) of the flavor source based on an output of a temperature detection element (a temperature detection element T1 or a temperature detection element T3) for detecting the temperature of the flavor source and to be able to change the power based on the temperature.

According to (4), for example, when a temperature of the flavor source changes during aerosol generation, power discharged to the first load is changed. Therefore, even when the temperature of the flavor source changes during the aerosol generation, the accuracy of controlling the discharging to the first load for generating the aerosol to which the desired amount of the flavor component is added can be improved throughout the aerosol generation.

(5) The power supply unit according to (4), further including:

a boosting circuit (a DC/DC converter 51) configured to be able to boost a voltage applied to the first load,

in which the processing device stops the boosting when the changed power can be discharged from the power supply to the first load even when boosting by the boosting circuit is stopped.

According to (5), after the power is changed, boosting in the boosting circuit is stopped, and then the changed power can be discharged to the first load. Therefore, it is possible to generate an aerosol having a desired amount of a flavor component while reducing power consumption associated with the boosting (a transition loss and a switching loss of the boosting circuit).

(6) The power supply unit according to (4), further including:

a boosting circuit (a DC/DC converter 51) configured to be able to boost a voltage applied to the first load; and a bypass circuit (a switch SW7) connected in parallel to the boosting circuit, in which the processing device causes the changed power to be discharged from the power supply to the first load by passing through the bypass circuit when the changed power can be discharged from the power supply to the first load even when boosting by the boosting circuit is stopped.

According to (6), after the power is changed, the changed power can be discharged to the first load by bypassing the boosting circuit. Therefore, it is possible to generate the aerosol having the desired amount of the flavor component while reducing power consumption associated with boosting and conduction (a transition loss, a switching loss, and a conduction loss of the boosting circuit).

(7) The power supply unit according to any one of (4) to (6),
in which the processing device is configured to determine power to be discharged from the power supply to the first load in response to the signal based on the determined power (the atomization power $P_{liquid}$'), a time for discharging the determined power ($t_{reach}$-$t_{start}$), the changed power ($P_{liquid}$), and a time for discharging the changed power ($t_{end}$-$t_{reach}$).

According to (7), when power discharged to the first load is changed during aerosol generation, the power to be discharged to the first load can be determined next after considering a changed content. Therefore, even when the power discharged to the first load is changed during the aerosol generation, the accuracy of controlling the discharging to the first load for generating the aerosol having the desired amount of the flavor component can be improved.

(8) The power supply unit according to (3),
in which the processing device does not acquire the output value and/or does not change the determined power while discharging the determined power to the first load.

According to (8), power discharged to the first load is not changed during the aerosol generation. Therefore, control can be stabilized as compared with a case where the power is changed during the aerosol generation.

(9) The power supply unit according to (1), further including:
a boosting circuit (a DC/DC converter 51) configured to be able to boost a voltage applied to the first load.

According to (9), power discharged to the first load can be increased by the boosting circuit. Therefore, compared with a case where no boosting circuit is provided, a range of the power discharged to the first load is widened, and aerosols having various desired amounts of flavor components can be generated.

(10) The power supply unit according to (9),
in which the processing device controls the boosting circuit so as not to perform the boosting when the determined power can be discharged from the power supply to the first load without being boosted by the boosting circuit.

According to (10), after the power is determined, the power can be discharged to the first load in a state where no boosting in the boosting circuit is performed. Therefore, it is possible to generate the aerosol having the desired amount of the flavor component while reducing the power consumption associated with the boosting (the transition loss and the switching loss of the boosting circuit).

(11) The power supply unit according to (10), further including:

a bypass circuit (a switch SW7) connected in parallel to the boosting circuit, in which the processing device causes the determined power to be discharged from the power supply to the first load by passing through the bypass circuit when the determined power can be discharged from the power supply to the first load without being boosted by the boosting circuit.

According to (11), after the power is determined, the power can be discharged to the first load by bypassing the boosting circuit. Therefore, it is possible to generate the aerosol having the desired amount of the flavor component while reducing the power consumption associated with the boosting and the conduction (the transition loss, the switching loss, and the conduction loss of the boosting circuit).

(12) A power supply unit for an aerosol inhaler that causes an aerosol generated from an aerosol source to pass through a flavor source to add a flavor component of the flavor source to the aerosol, the power supply unit including:

a power supply configured to be dischargeable to a first load that can atomize the aerosol source by consuming power; and a processing device configured to cause determined power to be discharged from the power supply to the first load in response to a signal from a sensor configured to output the signal indicating an aerosol generation request,

in which the processing device is configured to determine the power based on a time of discharging and a variable different from the time in discharging from the power supply to the first load in response to the signal before a previous time.

(13) A power supply unit (a power supply unit 10) for an aerosol inhaler (an aerosol inhaler 1) that causes an aerosol generated from an aerosol source (an aerosol source 22) to pass through a flavor source (a flavor source 33) to add a flavor component of the flavor source to the aerosol, the power supply unit including:

a power supply (a power supply 12) configured to be dischargeable to a first load (a first load 21) configured to heat the aerosol source; and
a processing device (an MCU 50) configured to cause determined power to be discharged from the power supply to the first load in response to a signal from a sensor (an intake sensor 15 or an operation unit 14) configured to output the signal indicating an aerosol generation request,
in which the processing device determines a first power (atomization power $P_{liquid}$) as power to be discharged to the first load when a temperature of the flavor source is equal to or higher than a target temperature ($T_{cap\_target}$), the temperature of the flavor source being acquired based on an output of a temperature detection element (a temperature detection element T1 or a temperature detection element T3) for detecting the temperature of the flavor source in response to the signal, and
in which the processing device determines a second power (atomization power $P_{liquid}'$) larger than the first power as power to be discharged to the first load when a temperature of the flavor source acquired in response to the signal is lower than the target temperature.

According to (13), the power discharged to the first load is controlled according to a temperature of the flavor source. Therefore, an aerosol to which a desired amount of a flavor component is added can be generated.

(14) A power supply unit for an aerosol inhaler that causes an aerosol generated from an aerosol source to pass through a flavor source to add a flavor component of the flavor source to the aerosol, the power supply unit including:

a power supply configured to be dischargeable to a first load that can atomize the aerosol source by consuming power; and
a processing device configured to cause determined power to be discharged from the power supply to the first load in response to a signal from a sensor configured to output the signal indicating an aerosol generation request,
in which the processing device determines a first power as power to be discharged to the first load when a temperature of the flavor source is equal to or higher than a target temperature, the temperature of the flavor source being acquired based on an output of a temperature detection element for detecting the temperature of the flavor source in response to the signal, and
in which the processing device determines a second power larger than the first power as power to be discharged to the first load when a temperature of the flavor source acquired in response to the signal is lower than the target temperature.

(15) An aerosol inhaler including:

the power supply unit according to any one of (1) to (12);
the aerosol source;
the flavor source;
the first load; and
the sensor.

(16) An aerosol inhaler including:

the power supply unit according to (13) or (14);
the aerosol source;
the flavor source;
the first load;
the sensor; and
the temperature detection element.

**Claims**

1. A power supply unit for an aerosol inhaler that causes an aerosol generated from an aerosol source to pass through a flavor source to add a flavor component of the flavor source to the aerosol, the power supply unit comprising:

   a power supply configured to be dischargeable to a first load configured to heat the aerosol source; and
   a processing device configured to cause determined power to be discharged from the power supply to the first load in response to a signal from a sensor configured to output the signal indicating an aerosol generation request, wherein the processing device is configured to determine the power based on a time of discharging and a variable different from the time in discharging from the power supply to the first load in response to the signal before a previous time.

2. The power supply unit according to claim 1,
   wherein the variable is power or electric energy discharged from the power supply to the first load.

3. The power supply unit according to claim 1 or 2,
   wherein the processing device is configured to acquire an output value of an element configured to output information on a temperature of the flavor source and use the output value as the variable.

4. The power supply unit according to claim 3,
   wherein while discharging the power to the first load, the processing device is configured to acquire a temperature of the flavor source based on an output of a temperature detection element for detecting the temperature of the flavor source and to be able to change the power based on the temperature.

5. The power supply unit according to claim 4, further comprising:

   a boosting circuit configured to be able to boost a voltage applied to the first load,
   wherein the processing device stops the boosting when the changed power can be discharged from the power supply to the first load even when boosting by the boosting circuit is stopped.

6. The power supply unit according to claim 4, further comprising:

   a boosting circuit configured to be able to boost a voltage applied to the first load; and
   a bypass circuit connected in parallel to the boosting circuit,
   wherein the processing device causes the changed power to be discharged from the power supply to the first load by passing through the bypass circuit when the changed power can be discharged from the power supply to the first load even when boosting by the boosting circuit is stopped.

7. The power supply unit according to any one of claims 4 to 6,
   wherein the processing device is configured to determine power to be discharged from the power supply to the first load in response to the signal based on the determined power, a time for discharging the determined power, the changed power, and a time for discharging the changed power.

8. The power supply unit according to claim 3,
   wherein the processing device does not acquire the output value and/or does not change the determined power while discharging the determined power to the first load.

9. The power supply unit according to claim 1, further comprising:
   a boosting circuit configured to be able to boost a voltage applied to the first load.

10. The power supply unit according to claim 9,
    wherein the processing device controls the boosting circuit so as not to perform the boosting when the determined power can be discharged from the power supply to the first load without being boosted by the boosting circuit.

11. The power supply unit according to claim 10, further comprising:

    a bypass circuit connected in parallel to the boosting circuit,
    wherein the processing device causes the determined power to be discharged from the power supply to the first load by passing through the bypass circuit when the determined power can be discharged from the power supply to the first load without being boosted by the boosting circuit.

12. An aerosol inhaler comprising:

the power supply unit according to any one of claims 1 to 11;
the aerosol source;
the flavor source;
the first load; and
the sensor.

*FIG.1*

*FIG.2*

*FIG.3*

*FIG.4*

FIG.5

# FIG.6

# FIG.7

FIG.8

## FIG.9

```
                    ┌──────────┐
                    │  START   │◄────────────┐
                    └──────────┘             │
 S0                      │                   │
  ◇─────────────────────────────────────┐    │
       IS POWER SUPPLY          NO       │    │
       TURNED ON?              ─────────────┘
  ◇────────────────────┐
 ┌───┐                 │ YES
 │ B │────────────────►│
 └───┘                 │                              S1
        ◇──────────────────────────────────────────────◇
          WHETHER IT IS FIRST SUCTION         NO
        AFTER POWER SUPPLY IS TURNED ON OR SECOND ────────────┐
          CARTRIDGE IS REPLACED?                              │
        ◇──────────────────────────────────────────────◇     │
                        │ YES          S3                  S2 │
 ┌────────────────────────────────────┐   ┌──────────────────────────────────────┐
 │ ACQUIRE Tcap_target SET WHEN PREVIOUS│   │ ACQUIRE TEMPERATURE OF FLAVOR SOURCE  │
 │ AEROSOL IS GENERATED AS INFORMATION  │   │ ACQUIRED BY TEMPERATURE DETECTION     │
 │ (Tcapsule) ON TEMPERATURE OF FLAVOR  │   │ ELEMENT AS INFORMATION (Tcapsule) ON  │
 │ SOURCE, AND SET ACQUIRED Tcap_target │   │ TEMPERATURE OF FLAVOR SOURCE, AND SET │
 │ AS TARGET TEMPERATURE Tcap_target OF │   │ ACQUIRED TEMPERATURE OF FLAVOR SOURCE │
 │ FLAVOR SOURCE                        │   │ AS TARGET TEMPERATURE Tcap_target OF  │
 └────────────────────────────────────┘   │ FLAVOR SOURCE                         │
                        │                   └──────────────────────────────────────┘
                        │◄──────────────────────────────────┐
 ┌────────────────────────────────────────────┐            │
 │ DETERMINE AEROSOL WEIGHT Waerosol REQUIRED  │            │
 │ TO ACHIEVE TARGET AMOUNT OF FLAVOR          │~S4         │
 │ COMPONENT Wflavor BASED ON Tcap_target AND  │            │
 │ FLAVOR COMPONENT REMAINING AMOUNT           │            │
 │ Wcapsule (npuff)                            │            │
 └────────────────────────────────────────────┘            │
                        │                                    │
 ┌──────────────────────────────┐                          │
 │ DETERMINE ATOMIZATION POWER   │~S5                       │
 │ Pliquid REQUIRED TO IMPLEMENT │                          │
 │ Waerosol                      │                          │
 └──────────────────────────────┘                          │
                        │        S6                          │
        ◇────────────────────────◇                         │
          IS Pliquid ≤ SECOND      NO                        │
          DEFAULT VALUE?          ──────────┐               │
        ◇────────────────────────◇          │        S7     │
 ┌───┐                  │ YES    S8          │  ┌────────────────────┐
 │ C │─────────────────►│                    └─►│ INCREASE Tcap_target│───┘
 └───┘        ┌─────────────────────────┐      └────────────────────┘
              │ ACQUIRE TEMPERATURE     │
              │ Tcap_sense OF FLAVOR    │
              │ SOURCE                  │
              └─────────────────────────┘
 S9                     │
 ┌─────────────────────────────────────┐
 │ CONTROL DISCHARGING TO SECOND LOAD   │
 │ BASED ON Tcap_sense and Tcap_target  │
 └─────────────────────────────────────┘
                        │
 S10                    │                          S11
        ◇───────────────────────◇          ◇───────────────────◇
          IS AEROSOL GENERATION   NO          IS NO              NO
          REQUEST DETECTED?      ──────────►  REQUEST DETECTED  ──────┐
        ◇───────────────────────◇          WITHIN PREDETERMINED      │
                        │ YES                  TIME?            S12   │
                        │              ◇───────────────────◇         │
                    ┌───────┐                  │ YES       ┌────────────────┐
                    │   A   │          ┌─────────────────┐ │ │      C       │
                    └───────┘          │ END DISCHARGING │ │ └────────────────┘
                                       │ TO SECOND LOAD  │ │
                                       └─────────────────┘ │
                                               │           │
                                       ┌─────────────────┐
                                       │ SHIFT TO SLEEP  │~S13
                                       │ MODE            │
                                       └─────────────────┘
```

32

*FIG.10*

A

STOP HEATING FLAVOR SOURCE
ACQUIRE TEMPERATURE Tcap_sense OF FLAVOR SOURCE — S14

S15

IS Tcap_sense ≥ Tcap_target? — NO

YES — S17

SUPPLY ATOMIZATION POWER Pliquid TO FIRST LOAD

S19

INCREASE ATOMIZATION POWER Pliquid AND SUPPLY INCREASED ATOMIZATION POWER Pliquid TO FIRST LOAD

S18

IS AEROSOL GENERATION REQUEST ENDED? — NO

YES

S20

IS AEROSOL GENERATION REQUEST ENDED? — NO

YES

STOP HEATING FIRST LOAD — S21

ACQUIRE SUPPLY TIME tsense OF ATOMIZATION POWER SUPPLIED TO FIRST LOAD — S22

S23

ADVANCE PUFF NUMBER COUNTER
npuff → npuff + 1

UPDATE FLAVOR COMPONENT REMAINING AMOUNT Wcapsule (npuff) BASED ON ATOMIZATION POWER, tsense, AND Tcap_target — S24

S25

IS Wcapsule (npuff) < REMAINING AMOUNT THRESHOLD? — NO

YES

GIVE NOTIFICATION PROMPTING REPLACEMENT OF SECOND CARTRIDGE — S26

S27

RESET PUFF NUMBER COUNTER TO "0"
INITIALIZE TARGET TEMPERATURE Tcap_target

S28

IS POWER SUPPLY TURNED OFF? — NO

B

YES

END

## FIG.11

## FIG.12

| Tcap_target | | | |
| Tcap_sense | | | |
| Pliquid | | | |

Tcap_target,Pliquid
SETTING COMPLETION

AEROSOL GENERATION
REQUEST DETECTION
tstart

END OF AEROSOL
GENERATION REQUEST
tend

SUPPLY TIME OF ATOMIZATION POWER
tsense

## FIG.13

Tcap_target

Tcap_sense

Pliquid'

Pliquid

Tcap_target,Pliquid
SETTING COMPLETION

AEROSOL GENERATION
REQUEST DETECTION
tstart

END OF AEROSOL
GENERATION REQUEST
tend

SUPPLY TIME OF ATOMIZATION POWER
tsense

## FIG.14

FIG.15

## FIG.16

## FIG.17

## FIG.18

## FIG.19

FIG.20

## FIG.21

*FIG.22*

# FIG.23

(A)

STOP HEATING FLAVOR SOURCE
ACQUIRE TEMPERATURE Tcap_sense OF
FLAVOR SOURCE — S14

S15

IS Tcap_sense ≥ Tcap_target? — NO

S19

INCREASE ATOMIZATION POWER Pliquid AND SUPPLY
INCREASED ATOMIZATION POWER Pliquid TO FIRST LOAD

(E) → YES — S17

SUPPLY ATOMIZATION POWER Pliquid TO FIRST LOAD

S18

IS AEROSOL GENERATION REQUEST ENDED? — NO

S20

IS AEROSOL GENERATION REQUEST ENDED? — NO

YES

STOP HEATING FIRST LOAD — S21

YES

ACQUIRE Tcap_sense — S31

ACQUIRE SUPPLY TIME tsense OF ATOMIZATION
POWER SUPPLIED TO FIRST LOAD — S22

S32

(E) ← YES — IS Tcap_sense ≥ Tcap_target?

S23

ADVANCE PUFF NUMBER COUNTER
npuff → npuff + 1

NO

UPDATE FLAVOR COMPONENT REMAINING AMOUNT
Wcapsule (npuff) BASED ON ATOMIZATION POWER, tsense,
AND Tcap_target — S24

S25

IS Wcapsule (npuff) < REMAINING
AMOUNT THRESHOLD? — NO

YES

GIVE NOTIFICATION PROMPTING REPLACEMENT
OF SECOND CARTRIDGE — S26

S27

RESET PUFF NUMBER COUNTER TO "0"
INITIALIZE TARGET TEMPERATURE Tcap_target

S28

IS POWER SUPPLY
TURNED OFF? — NO

YES

(B)

END

## FIG.24

Europäisches
Patentamt

European
Patent Office

Office européen
des brevets

# EUROPEAN SEARCH REPORT

Application Number

EP 21 15 9231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/042251 A1 (YAMADA MANABU [JP] ET AL) 16 February 2017 (2017-02-16) | 1,2,9, 10,12 | INV. A24F40/50 A24F40/57 |
| Y | * figures 1-3 * | 11 | |
| A | * paragraph [0058] - paragraph [0059] * <br> * paragraph [0100] * <br> * paragraph [0114] * | 3-8 | |
| X | US 2019/307166 A1 (MATSUMOTO HIROFUMI [JP] ET AL) 10 October 2019 (2019-10-10) | 1,9,10, 12 | |
| Y | * figures 1-5 * | 11 | |
| A | * paragraph [0074] * <br> * paragraph [0114] * <br> * paragraph [0254] * | 3-8 | |
| X | US 2019/335816 A1 (YAMADA MANABU [JP] ET AL) 7 November 2019 (2019-11-07) <br> * figure 1B * <br> * paragraph [0125] - paragraph [0128] * | 1,12 | |
| X | CA 3 079 163 A1 (JAPAN TOBACCO INC [JP]) 25 April 2019 (2019-04-25) <br> * figures 1,2 * <br> * paragraph [0089] * <br> * paragraph [0138] * | 1,12 | TECHNICAL FIELDS SEARCHED (IPC) <br><br> A24F |
| Y | US 2006/006850 A1 (INOUE MANABU [JP] ET AL) 12 January 2006 (2006-01-12) <br> * figure 1 * <br> * abstract * | 11 | |
| A | US 2018/360122 A1 (AOUN WALID ABI [GB] ET AL) 20 December 2018 (2018-12-20) <br> * figures 2,3 * <br> * paragraph [0056] * <br> * paragraph [0062] * | 1-12 | |

The present search report has been drawn up for all claims

| Place of search | Date of completion of the search | Examiner |
|---|---|---|
| Munich | 30 June 2021 | Martin, Raynald |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 15 9231

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2021

| Patent document cited in search report | Publication date | Patent family member(s) | Publication date |
|---|---|---|---|
| US 2017042251 A1 | 16-02-2017 | CA 2947135 A1 | 05-11-2015 |
| | | CN 106231936 A | 14-12-2016 |
| | | EA 201692211 A1 | 28-02-2017 |
| | | EP 3138424 A1 | 08-03-2017 |
| | | ES 2745200 T3 | 28-02-2020 |
| | | JP 6167228 B2 | 19-07-2017 |
| | | JP 6789592 B2 | 25-11-2020 |
| | | JP 6840186 B2 | 10-03-2021 |
| | | JP 2017192393 A | 26-10-2017 |
| | | JP 2019150034 A | 12-09-2019 |
| | | JP WO2015166952 A1 | 20-04-2017 |
| | | KR 20160137627 A | 30-11-2016 |
| | | TW 201605363 A | 16-02-2016 |
| | | US 2017042251 A1 | 16-02-2017 |
| | | WO 2015166952 A1 | 05-11-2015 |
| US 2019307166 A1 | 10-10-2019 | AU 2015347931 A1 | 29-06-2017 |
| | | CN 107072319 A | 18-08-2017 |
| | | CN 110477458 A | 22-11-2019 |
| | | EA 201791039 A1 | 29-09-2017 |
| | | EP 3207811 A1 | 23-08-2017 |
| | | EP 3560365 A1 | 30-10-2019 |
| | | EP 3632243 A1 | 08-04-2020 |
| | | ES 2788477 T3 | 21-10-2020 |
| | | JP 6461190 B2 | 30-01-2019 |
| | | JP 6461421 B1 | 30-01-2019 |
| | | JP 6545348 B2 | 17-07-2019 |
| | | JP 6649523 B2 | 19-02-2020 |
| | | JP 2019050809 A | 04-04-2019 |
| | | JP 2019050815 A | 04-04-2019 |
| | | JP 2019193642 A | 07-11-2019 |
| | | JP WO2016076178 A1 | 13-07-2017 |
| | | KR 20170076768 A | 04-07-2017 |
| | | KR 20190076066 A | 01-07-2019 |
| | | KR 20190110138 A | 27-09-2019 |
| | | US 2017238606 A1 | 24-08-2017 |
| | | US 2019307166 A1 | 10-10-2019 |
| | | WO 2016075747 A1 | 19-05-2016 |
| | | WO 2016076178 A1 | 19-05-2016 |
| US 2019335816 A1 | 07-11-2019 | CA 3048780 A1 | 02-08-2018 |
| | | CN 108882750 A | 23-11-2018 |
| | | EA 201991318 A1 | 25-02-2020 |
| | | EP 3545780 A1 | 02-10-2019 |
| | | JP 6644157 B2 | 12-02-2020 |
| | | JP WO2018138751 A1 | 31-01-2019 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.      EP 21 15 9231

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2021

| Patent document cited in search report | Publication date | Patent family member(s) | Publication date |
|---|---|---|---|
| | | KR 20190103316 A | 04-09-2019 |
| | | US 2019335816 A1 | 07-11-2019 |
| | | WO 2018138751 A1 | 02-08-2018 |
| CA 3079163 A1 | 25-04-2019 | CA 3079163 A1 | 25-04-2019 |
| | | CN 111246757 A | 05-06-2020 |
| | | EA 202090964 A1 | 14-07-2020 |
| | | EP 3698656 A1 | 26-08-2020 |
| | | JP WO2019077710 A1 | 02-07-2020 |
| | | KR 20200068707 A | 15-06-2020 |
| | | TW 201917404 A | 01-05-2019 |
| | | US 2020237011 A1 | 30-07-2020 |
| | | WO 2019077710 A1 | 25-04-2019 |
| US 2006006850 A1 | 12-01-2006 | CN 1701482 A | 23-11-2005 |
| | | EP 1543599 A1 | 22-06-2005 |
| | | JP 4177412 B2 | 05-11-2008 |
| | | JP 2007529185 A | 18-10-2007 |
| | | US 2006006850 A1 | 12-01-2006 |
| | | WO 2005013455 A1 | 10-02-2005 |
| US 2018360122 A1 | 20-12-2018 | AU 2015334902 A1 | 27-04-2017 |
| | | AU 2019200330 A1 | 07-02-2019 |
| | | AU 2021203342 A1 | 24-06-2021 |
| | | BR 112017008315 A2 | 20-02-2018 |
| | | CA 2963957 A1 | 28-04-2016 |
| | | CA 3078511 A1 | 28-04-2016 |
| | | CN 107072317 A | 18-08-2017 |
| | | EP 3209151 A1 | 30-08-2017 |
| | | EP 3440950 A1 | 13-02-2019 |
| | | JP 6720162 B2 | 08-07-2020 |
| | | JP 6845190 B2 | 17-03-2021 |
| | | JP 2017538398 A | 28-12-2017 |
| | | JP 2018201514 A | 27-12-2018 |
| | | KR 20170073664 A | 28-06-2017 |
| | | KR 20190003832 A | 09-01-2019 |
| | | KR 20210049976 A | 06-05-2021 |
| | | RU 2647252 C1 | 14-03-2018 |
| | | RU 2018106929 A | 26-08-2019 |
| | | US 2017347706 A1 | 07-12-2017 |
| | | US 2018360122 A1 | 20-12-2018 |
| | | WO 2016062777 A1 | 28-04-2016 |

EPO FORM P0459

For more details about this annex : see Official Journal of the European Patent Office, No. 12/82

**EP 3 871 524 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017511703 T
- WO 2018017654 A
- WO 2018020619 A